# EUROPEAN PATENT SPECIFICATION

(11) **EP 2 149 605 B1**
(45) Date of publication and mention of the grant of the patent: **03.07.2013**
(21) Application number: 09169826.6
(22) Date of filing: 19.03.2008
(51) Int. Cl.: C12N 15/113, C12N 15/11

(54) **Short RNA antagonist compounds for the modulation of target mRNA**
Kurze RNA Antagonistverbindungen zur Modulation von Ziel mRNA
Composés ARN antagonistes courts pour la modulation de l'ARNm cible

(30) Priority: 22.03.2007 US 896419; 30.08.2007 US 969016; 04.10.2007 US 977409; 26.11.2007 US 990125; 17.09.2007 US 972932; 03.12.2007 US 992050; 07.12.2007 US 12191; 07.12.2007 US 12185; 24.01.2008 US 23244; 24.01.2008 US 23250; 09.10.2007 WO PCT/EP2007/060703
(43) Date of publication of application: 03.02.2010
(62) Divisional of application: 08718035.2
(73) Proprietor: Santaris Pharma A/S, 2970 Hørsholm (DK)
(72) Inventor: Hansen, Jens Bo, 2920, Charlottenlund (DK); Orum, Henrik, 3500, Værløse (DK); Hansen, Henrik, 2610, Roedovre (DK); Straarup, Ellen Marie, 3460, Birkerød (DK); Nielsen, Niels Fisker, 2800 Kgs. Lyngby (DK); Hedtjärn, Maj, 2450, Copenhagen SV (DK)
(74) Representative: Turner, Mark Frederic Paris

(56) References cited:
- WO-A-03/085110
- WO-A-2006/050734
- KURRECK J ET AL: "Design of antisense oligonucleotides stabilized by locked nucleic acids" NUCLEIC ACIDS RESEARCH, OXFORD UNIVERSITY PRESS, SURREY, GB, vol. 30, no. 9, 1 May 2002 (2002-05-01), pages 1911-1918, XP002281375 ISSN: 0305-1048

## Description

### FIELD OF THE INVENTION

The present invention provides antisense oligonucleotides (oligomers), compositions and methods for modulating the expression of target mRNAs. In a particularly interesting aspect, the invention provides LNA gapmer oligomers, which have a total of between 1 and three, such as one or two phosphodiester internucleoside linkages. Such oligomers have been found to have superior bioavailability and have also been found to selectively accumulate in kidney cells.

### BACKGROUND

Zhou and Agrawal et al, Bioorganic & Medicinal Chemistry Letters 8 (1998) 3269-3274, reports on 2'-O methylribonucleoside gapmer phosphorothioate oligonucleotides, which have flanks which each comprise four and five 2'-0 methylribonucleoside units, where each flank comprises two phosphodiester bonds. The introduction of phosphodiester bonds was found to result in intravenous distribution patterns similar to the fully phosphorothioate molecules, but have reduced side effects.

### SUMMARY OF THE INVENTION

The invention provides for an oligomer consisting of a contiguous nucleobase sequence of a total of between 10 - 20 nucleobases, such as 11, 12, 13, 14, 15, 16, 17, 18, 19 nucleobase units, wherein the contiguous nucleobase sequence is of formula (5' - 3'), A-B-C, or optionally A-B-C-D or D-A-B-C, wherein;
- region A (5' region) consists or comprises 1-6 LNA units, preferably between 2-5 nucleotide analogues, such as 2-5 LNA units, such as 3 or 4 nucleotide analogues, such as 3 or 4 LNA units and;
- region B (central domain), immediately 3' (i.e. contiguous) to A, consists of between 6-12 contiguous nucleobase units which are capable of recruiting RNAseH when formed in a duplex with a complementary RNA molecule (such as a mRNA target) such as DNA units; preferably region B comprises of at least one DNA sugar unit, such as 1-12 DNA units, preferably between 4-12 DNA units, more preferably region B consists of between 6-10 DNA units, such as between 7-10 DNA units, most preferably 8, 9 or 10 DNA units, and;
- region C(3' region) immediately 3' to B, consists or comprises 1-6 LNA units such as between 2-5 LNA units, such as 3 or 4 LNA units; and
- region D, when present, consists of one or two DNA units
wherein said oligomer comprises of a total of 1 or 2 phosphodiester linkages; wherein the phosphodiester bond or bonds are inserted between or adjacent to nucleotide analogue(s) of region A and/or C, and
wherein all the remaining internucleoside linkages are phosphorothioate.

The invention provides for an oligomer consisting of a contiguous nucleobase sequence of a total of 10, 11, 12, 13, 14, 15 or 16 nucleobase units, wherein the contiguous nucleobase sequence is of formula (5' - 3'), A-B-C, or optionally A-B-C-D or D-C-B-A, wherein:
A consists of 1, 2 or 3 LNA units;
B consists of 7, 8, or 9 contiguous nucleobase units which are capable of recruiting RNAseH when formed in a duplex with a complementary RNA molecule (such as a mRNA target); and
C consists of 1, 2 or 3 LNA units; and
D, where present, consists of 1 DNA unit.

Wherein or Wherein, the oligomer comprises a total of one or two phosphodiester bonds. INS: Wherein the phosphodiester bond or bonds are inserted between or adjacent to nucleotide analogues of region A and/or C, and the remaining internucleoside linkages are phosphorothioate linkages.

The invention provides for an oligomer consisting of a contiguous nucleobase sequence of a total of 10, 11, 12, 13 or 14 nucleobase units, wherein the contiguous nucleobase sequence is of formula (5' - 3'), A-B-C, or optionally A-B-C-D or D-C-B-A, wherein; region A consists of 1, 2 or 3 LNA units; region B consists of 7, 8 or 9 contiguous nucleobase units which are capable of recruiting RNAseH when formed in a duplex with a complementary RNA molecule (such as a mRNA target); and region C consists of 1, 2 or 3 LNA units; and wherein said * oligomer comprises of a total of 1 or 2 phosphodiester linkages and the remaining internucleoside linkages are phosphaothioate and region D, when present, consists of one DNA unit.

The invention further provides a conjugate comprising the oligomer according to the invention, such as a conjugate which, in addition to the nucleobase sequence of the oligomer comprises at least one non-nucleotide or non-polynucleotide moiety covalently attached to the oligomer of the invention.

The invention provides for pharmaceutical composition comprising the oligomer or conjugate of the invention, and a pharmaceutically acceptable diluent, carrier, salt or adjuvant.

The invention further provides for an oligomer according to the invention, for use in medicine.

The invention further provides for the use of the oligomer of the invention for the manufacture of a medicament for the treatment of one or more of the diseases referred to herein.

The invention further provides for an oligomer according to the invention, for use for the treatment of one or more of the diseases referred to herein.

The oligomer of the invention may be used in a method for the treatment of one or more of the diseases referred to herein in a patient suffering from said disease, said method comprising administering the oligomer, conjugate or pharmaceutical of the invention to said patient.

The invention provides for an in vitro method of inhibiting or reducing the level of a A target mRNA in a cell or a tissue, the method comprising the step of contacting said cell or tissue with on oligomer, a conjugate, or a pharmaceutical composition according to the invention so that level of target mRNA is inhibited or reduced.

The invention provides for an in vitro method of inhibiting or reducing the level of a target protein in a cell or a tissue, the method comprising the step of contacting said cell or tissue with on oligomer, a conjugate, or a pharmaceutical composition according to the invention so that level of target protein is inhibited or reduced. In one embodiment the target protein is Hif1alpha.

The invention provides for an in vitro method for the reduction in the cellular concentration of a mRNA in a mammalian cell, said method comprising the administration of an oligomer, or the conjugate according to the invention, to the mammalian cell, wherein said mammalian cell comprises an mRNA species which comprises a nucleobase sequence which is complementary to said oligomer.

The invention provides for a method for modifying a fully phosphorothioate oligomer to enhance its accumulation or bioavailability in an organ, said method comprising replacing a total of one or two of the phosphorothioate linkages with phosphodiester bonds, wherein said oligomers consist of a contiguous nucleobase sequence A-B-C, or A-B-C-D, or D-A-B-C, as defined in the claims, INS: Wherein the phosphodiester bond or bonds are inserted between or adjacent to nucleotide analogues of region A and/or C.

The invention provides for a method for modifying a fully phosphorothioate oligomer to enhance its accumulation or bioavailability in an organ, said method comprising replacing a total of one or two of the phosphorothioate linkages with phosphodiester bonds, wherein said oligomers consisting of a contiguous nucleobase sequence of a total of 10 - 20 nucleobase units, such as 11, 12, 13, 14, 15, 16, 17, 18, 19 or 20, nucleobase units wherein the contiguous nucleobase sequence is of formula (5' - 3'), A-B-C, A-B-C-D or D-A-B-C; wherein region A (5' region) consists or comprises of, 1-6 LNA units, such as 2, 3, 4 or 5 LNA units, such as 3 or 4 LNA units and; region B (central domain), preferably immediately 3' (*i*.*e*. contiguous) to A, consists or comprises at least one DNA sugar unit, such as 1-12 DNA units, preferably between 4-12 DNA units, more preferably between 6-10 DNA units, such as between 7-10 DNA units, most preferably 8, 9 or 10 DNA units, and; region C(3' region) immediately 3' to B, consists or comprises at of at 1-6 LNA units, such as 2, 3, 4 or 5 LNA units, such as 3 or 4 LNA units, and, when present region D consists of 1, 2 or 3 nucleotide units, such as DNA. The phosphodiester bonds are inserted between or adjacent to nucleotide analogue(s) of region A and/or C. In one embodiment region B consists of 11 nucleobases, such as DNA units.

In one embodiment, the oligomer targets Hif-1alpha mRNA (e.g. SEQ ID NO 66).

In one embodiment, the oligomeric compound consists of a total of 10-15 or 10 - 16 nucleobases, wherein the nucleobase sequence of said compound corresponds to a contiguous sub-sequence present in the hif1-alpha mRNA (e.g. SEQ ID NO 66), wherein said compound comprises at least 2 or at least 3 nucleotide analogues.

In one embodiment, the oligomer consists of a total of 10-15, or 10 - 16 nucleobases, wherein the nucleobase sequence of said compound corresponds to a contiguous sub-sequence present in SEQ ID NO 51, 52, 53, 54 or 55, wherein said compound comprises at least 2 or at least 3 nucleotide analogues.

The invention provides for an in vitro method of down-regulating hif-1alpha in a cell, said method comprising administering the oligomer or conjugate according to the invention to a cell which is expressing Hif1-alpha so that the expression of hif-1alpha in the cell is reduced.

The invention further provides for the use of the oligomer targeting Hif-1alpha, or a conjugate thereof, for the manufacture of a medicament for the treatment of cancer.

The invention further provides for the oligomer targeting Hif-1alpha, or a conjugate thereof, as a medicament, such as a medicament for the treatment of cancer.

The oligomer of invention may be used in a method for treating cancer, said method comprising administering the use of the oligomer targeting Hif-1alpha, or a conjugate or a pharmaceutical composition which comprises said oligomer to a patient in need thereof (typically one suffering from cancer).

### BRIEF DESCRIPTION OF THE FIGURES

**Figure 1****.** The apoB-100 mRNA expression was measured by qPCR normalized to the house keeping gene GAPDH and presented relative to the saline group.
Mice (n = 3 or 5) were dosed 3 consecutive days and sacrifice 24 hours after the last dosing, liver was isolated and analyzed.
**Figure 2****.** Serum cholesterol levels at sacrifice (day 3) after dosing oligos of different length.
**Figure 3****.** The apoB-100 mRNA expression was measured by qPCR normalized to the house keeping gene GAPDH and presented relative to the saline group.
Mice (n = 5) were dosed once and sacrifice different days after dosing, liver was isolated and analyzed.
**Figure 4****.** Serum total cholesterol measured at sacrifice (days 1, 3, 5, 6 and 8) using a ABX pentra kit., n = 5.
**Figure 5****.** The apoB-100 mRNA expression was measured by qPCR normalized to the house keeping gene GAPDH and presented relative to the saline group. Mice (n = 5) were dosed once and sacrifice different days after dosing, liver was isolated and analyzed.
**Figure 6****.** Serum total cholesterol measured at sacrifice (days 1, 3, 5, 6, 8 and 16) using a ABX pentra kit., n = 5.
**Figure 7****.** The apoB-100 mRNA expression was measured by qPCR normalized to the house keeping gene GAPDH and presented relative to the saline group. Mice (n = 5) were dosed 1 or 5 mg/kg 3 consecutive days and sacrificed 24 hours after last dosing (day 3), liver was isolated and analyzed.
**Figure 8****.** Serum total cholesterol measured at sacrifice (day 3) using a ABX pentra kit., n = 5.
**Figure 9****.** The effect of dosing three doses at 5 mg/kg/dose of oligos of different length on ApoB-100 mRNA expression.
**Figures 10** **and** **11****.** SEQ ID NOS 16, 41, 17, 26 , 34 all against ApoB mRNA down regulated ApoB 30%, 30%, 80%, 90%, 90% respectively but had no effect on Hif1-alpha mRNA whereas SEQ ID NOs 50, 51 and 52 against Hif1-alpha down-regulated Hif1-alpha mRNA by 0%, 40% and 70% respectively but had no effect on ApoB mRNA.
**Figure 12****.** SEQ ID NOS 16, 41, 17 , 26 , 34 all against ApoB mRNA down regulated serum cholesterol by 40%, 40%, 80%, 90%, 85% respectively whereas SEQ ID NO's 50, 51, 52 showed no effect on serum cholesterol.
**Figure 13** NMRI mice were dosed 5 mg/kg/dose on 3 consecutive days (one dose/day i.v.) and animals were sacrificed 24 hours after last dosing. At sacrifice, liver tissue were sampled. RNA was isolated from the tissues and the expression of Hif1-alpha mRNA was measured using qPCR.
**Figure 14** NMRI mice were dosed 5 mg/kg/dose on 3 consecutive days (one dose/day i.v.) and animals were sacrificed 24 hours after last dosing. At sacrifice, kidney tissue were sampled. RNA was isolated from the tissues and the expression of Hif1-alpha mRNA was measured using qPCR.
**Figure 15** Female C57BL/6 mice were dosed with either 0.2 mg/kg or 1 mg/kg on 3 consecutive days (one dose/day i.v.) and animals were sacrificed 24 hours after last dosing. At sacrifice, liver and kidney tissue were sampled. RNA was isolated from the tissues and the expression of ApoB mRNA was measured using qPCR.
**Figure 16****:** The amount of oligomer SEQ ID NO 67 present in the urine of mouse injected with 1 X 50mg/kg at 1hr, 6hr, and 24hrs after injection, and the total amount.
**Figure 17****:** The amount of oligomer SEQ ID NO 68 present in the urine of mouse injected with 1 X 50mg/kg at 1hr, 6hr, and 24hrs after injection, and the total amount.
**Figure 18****:** The amount of oligomers SEQ ID NO 67 and SEQ ID No 68 present in the liver and kidney of mice injected with 1 X 50mg/kg at 24hrs after injection.
**Figure 19****:** Biodistribution/bioavailability of oligomers SEQ ID NO 67 and SEQ ID No 68 present in the liver, kidney, urine and other tissues of mice injected with 1 X 50mg/kg at 24hrs after injection.

### DETAILED DESCRIPTION OF THE INVENTION

The present invention provides oligomeric compounds (oligomers), compositions, and methods, for modulating the expression of target mRNAs. In a particularly interesting aspect, the invention provides LNA gapmer oligomers of . Such according to claim 1. oligomers have been found to have superior bioavailability and have also been found to selectively accumulate in kidney cells.

Oligomers with the A-B-C or A-B-C-D or D-A-B-C design are referred to as gapmers herein. Gapmers of between 10 - 14 nucleobases are also referred to as shortmers. Suitably, the length of the oligomer (contiguous nucleobase sequence) may be 10, 11, 12, 13, 14, 15, 16, 17, 18, 19, or 20 nucleobases in length. An a preferred embodiment, oligomer of the invention consists or comprises of a contiguous nucleobase sequence of (a total of) between 10 and 16, such as 15 or 16 nucleobases, or 10 and 14 nucleobases, such as 11, 12, and 13 nucleobases, and preferably between 10 and 13 nucleobases.

In one embodiment, the oligomer of the invention consists of a nucleobase sequence with is 100% complementary to a corresponding region of the target mRNA.

In one embodiment, the terms "oligomeric compound" or "oligomer", which are used interchangeably, refer to an oligonucleotide (which may comprise nucleotides and nucleotide analogues) which can induce a desired therapeutic effect in humans through for example binding by hydrogen bonding to a target nucleic acid. It is also envisaged that the oligomeric compounds disclosed herein may have non-therapeutic applications, such as diagnostic applications. Then oligomer is a single stranded (antisense) oligonucleotide.

In one embodiment, the oligomer of the invention does not comprise RNA units.

The oligomeric compounds may hybridize to any site along the target mRNA nucleic acid, such as the 5' untranslated leader, exons, introns and 3 'untranslated tail. However, it is preferred that the oligonucleotides hybridise to the mature mRNA form of the target nucleic acid.

When designed as an antisense inhibitor, for example, the oligonucleotides of the invention bind to the target nucleic acid and modulate the expression of its cognate protein. Preferably, such modulation produces an inhibition of expression of at least 10% or 20% compared to the normal expression level, more preferably at least a 30%, 40%, 50%, 60%, 70%, 80%, 90% or 95% inhibition compared to the normal expression level. Suitably, such modulation is seen when using between 5 and 25nM concentrations of the compound of the invention. In the same of a different embodiment, the inhibition of expression is less than 100%, such as less than 98% inhibition, less than 95% inhibition, less than 90% inhibition, less than 80% inhibition, such as less than 70% inhibition. Modulation of expression level is determined by measuring protein levels, e.g. by the methods such as SDS-PAGE followed by western blotting using suitable antibodies raised against the target protein. Alternatively, modulation of expression levels can be determined by measuring levels of mRNA, eg. by northern blotting or quantitative RT-PCR. When measuring via mRNA levels, the level of down-regulation when using an appropriate dosage, such as between 5 and 25nM concentrations, is, in one embodiment, typically to a level of between 10-20% the normal levels in the absence of the compound of the invention.

The terms "corresponding to" and "corresponds to" refer to the comparison between the nucleobase sequence of the oligomer (the contiguous nucleobase sequence) and the equivalent nucleotide sequence of i) the reverse complement of the nucleic acid target, such as the mRNA which encodes the target protein (e.g. Hif-1alpha), and/or ii) the sequence of nucleotides provided herein such as the group consisting of SEQ ID NOS: 51-55. Nucleotide analogues are compared directly to their equivalent or corresponding nucleotides. Therefore, in one embodiment, the terms "corresponding to"/ "corresponds to" refer to the comparison between the combined sequence of nucleotides and nucleotide analogues of the oligomeric compound of the invention, or subsequence thereof, and the equivalent nucleotide sequence of Apolipoprotein B or Hif1alpha nucleic acid sequence (i.e. the nucleic acid target).

Nucleotide analogues are compared directly to their equivalent or corresponding nucleotides.

The terms "corresponding nucleotide analogue" and "corresponding nucleotide" are intended to indicate that the nucleobase in the nucleotide analogue and the nucleotide are identical. For example, when the 2-deoxyribose unit of the nucleotide is linked to an adenine, the "corresponding nucleotide analogue" contains a pentose unit (different from 2-deoxyribose) linked to an adenine.

The term "nucleobase" is used as a collective term which encompasses both nucleotides and nucleotide analogues. A nucleobase sequence is a sequence which comprises at least two nucleotides or nucleotide analogues. In one embodiment the nucleobase sequence may comprise of only nucleotides, such as DNA units, in an alternative embodiment, the nucleobase sequence may comprise of only nucleotide analogues, such as LNA units.

The term "nucleic acid" is defined as a molecule formed by covalent linkage of two or more nucleotides.

The terms "nucleic acid" and "polynucleotide" are used interchangeable herein.

The term "target nucleic acid", as used herein refers to the DNA or RNA sequence encoding the mammalian target polypeptide (target for down-regulation). In one embodiment, for example when used in research or diagnostics the "target nucleic acid" may be a cDNA or a synthetic oligonucleotide derived from the above DNA or RNA nucleic acid targets. The oligomer according to the invention is preferably capable of hybridising to the target nucleic acid.

The term "at least one" comprises the integers larger than or equal to 1, such as 1, 2, 3, 4, 5, 6, 7, 8, 9, 10, 11, 12, 13, 14, 15, 16, 17, 18, 19, 20 and so forth.

### Target Sequences

The target of the oligomers of the invention is typically a mRNA which is comprises a nucleotide sequence which is complementary (or essentially complementary - *e*.*g*. may, in one embodiment comprise one or two mismatches) to the contiguous nucleobase sequence of the oligomer of the invention - *i*.*e*. the nucleotide region of the target 'corresponds' to the contiguous nucleobase sequence of the oligomer.

In one embodiment the target mRNA is a mammalian mRNA, such as a human mRNA, selected form the group consisting of ApoB-100 or Hif-1alpha, (see SEQ ID NO 66 for human hif 1 alpha sequence - Genbank accession number NM_001530), and naturally occurring allelic variants and homologues thereof. In the tables of oligonucleotides herein, which include specific oligomers according to the invention - regions A and C are in bold, regions B and where present D, are not in bold. A superscript ^{m} prior to the base letter C, refers to methyl-cytosine; a subscript ₛ after the base letter, refers to a phosphorothioate linkage; a superscript ^{o} after the base letter refers to oxy-LNA, particularly beta-D-oxy-LNA. It should be noted that that, in one embodiment, other LNA monomers as disclosed herein, may be used in place of oxy-LNA. It should be noted that that, in one embodiment, non-methylated LNA cytosine monomer may be used.

**ApoB-100 -** see US 60/896,419 US60/896,419 provides the medical disorders associated with abnormal Apo-B100 levels which may be treated with oligomers of the invention targeting Apo-B100. See the examples for oligomers targeting ApoB.

**Hif-1alpha** - WO2006/050734-medical disorders associated with abnormal Hif-1alpha levels which may be treated with oligomers of the invention targeting Hif-1alpha. Oligomers targeting Hif-1alpha include:

| | | | |
|---|---|---|---|
| SEQ ID NO:41 | 14 | 2-9-2 | **5'-GG**caagcatcc**TG**t**-3'** |
| SEQ ID NO:42 | 14 | 2-8-3 | **5'-GG**caagcatc**CTG**t**-3'** |
| SEQ ID NO:43 | 14 | 3-7-3 | **5'-GGC**aagcatc**CTG**t**-3'** |
| SEQ ID NO:44 | 13 | 2-9-2 | **5'-GG**caagcatcc**TG-3'** |
| SEQ ID NO:45 | 13 | 2-8-3 | **5'-GG**caagcatg**CTG-3'** |
| SEQ ID NO:46 | 13 | 2-8-3 | **5'-GC**aagcatcc**TGT-3'** |
| SEQ ID NO:47 | 13 | 2-9-2 | **5'-GC**aagcatcct**GT-3'** |
| SEQ ID NO:48 | 12 | 1-9-2 | **5'-G**caagcatcc**TG-3'** |
| SEQ ID NO:49 | 12 | 2-8-2 | **5'-GC**aagcatcc**TG-3'** |
| SEQ ID NO:50 | 12 | 2-7-3 | **5'-GC**aagcatc**CTG-3'** |
| SEQ ID NO:51 | **5'-GG**CAAGCATCC**TG**T-**3'** | | |
| SEQ ID NO:52 | **5'-GG**CAAGCATCC**TG-3'** | | |
| SEQ ID NO:53 | **5'-GC**AAGCATCC**TGT-3'** | | |
| SEQ ID NO:54 | **5'-G**CAAGCATCC**TG**-**3'** | | |
| SEQ ID NO:55 | TGGCAAGCATCCTGTA | | |

Hif-1a oligomer sequence motifs. In one embodiment the oligomeric compound of the invention has a sequence motif (contiguous nucleobase sequence) which is found within or is identical to any one of SEQ ID 41 - 55.

### Specific HIF-1a targeting compounds

| **Test substance** | **Sequence** | **Size** |
|---|---|---|
| SEQ ID NO: 56 | 5'- **Tₛ^{o} Gₛ^{o} Gₛ^{o}** cₛ aₛ aₛ gₛ cₛ aₛ tₛ cₛ cₛ **Tₛ^{o} Gₛ^{o} Tₛ^{o}** a -3' | 16 |
| SEQ ID NO: 57 | 5'- **Gₛ^{o} Gs^{o}** cₛ aₛ aₛ gₛ cₛ aₛ tₛ cₛ cₛ **Tₛ^{o} Gₛ^{o}** t -3' | 14 |
| SEQ ID NO: 58 | 5'- **Gₛ^{o} Cₛ^{o}** aₛ aₛ gₛ cₛ aₛ tₛ cₛ cₛ **Tₛ^{o} G^{o}** -3' | 12 |
| SEQ ID NO: 59 | 5'- **Gₛ^{o} Gₛ^{o m} Cₛ^{o}** aₛ aₛ gₛ cₛ aₛ tₛ cₛ cₛ **Tₛ^{o} Gₛ^{o} T^{o}** -3' | 14 |
| SEQ ID NO: 60 | 5'- **Gₛ^{o} Gₛ^{o}** cₛ aₛ aₛ gₛ cₛ aₛ tₛ cₛ cₛ **Tₛ^{o} Gₛ^{o} T^{o}** -3' | 14 |
| SEQ ID NO: 61 | 5'- **Gₛ^{o} Gₛ^{o}** cₛ aₛ aₛ gₛ cₛ aₛ tₛ cₛ cₛ **Tₛ^{o} G^{o}** -3' | 13 |
| SEQ ID NO: 62 | 5'- **Gₛ^{o} Gₛ^{o}** cₛ aₛ aₛ gₛ cₛ aₛ tₛ cₛ **^{m}Cₛ^{o} Tₛ^{o} G^{o}** -3' | 13 |
| SEQ ID NO: 63 | 5'- **Gₛ^{o m}Cₛ^{o}** aₛ aₛ gₛ cₛ aₛ tₛ Cₛ Cₛ **Tₛ^{o} Gₛ^{o} T^{o}** -3' | 13 |
| SEQ ID NO: 64 | 5'- **Gₛ^{o m}Cₛ^{o}** aₛ aₛ gₛ cₛ aₛ tₛ cₛ cₛ tₛ **Gₛ^{o} T^{o}** -3' | 13 |
| SEQ ID NO: 65 | 5'- **Gₛ^{o}** cₛ aₛ aₛ gₛ cₛ aₛ tₛ cₛ cₛ **T^{o} G^{o}** -3' | 12 |

In one embodiment, the oligomer of the invention is SEQ ID NO 69. In one embodiment the nucleobase sequence selected from the group consisting of SEQ ID NOS 51, 52, 53, 54, or 55 (motif sequences), or a corresponding subsequence thereof.

In one embodiment, the oligomeric compound of the invention consists of a contiguous nucleobase sequence of a total of 10-14 nucleobases in length.

In one embodiment, the contiguous nucleobase sequence consists of a total of 10, 11, 12, 13 or 14 nucleobase units, preferably 10, 11, 12 or 13, present in, or corresponding to (such as the same base (A,T,C or G) sequence) the nucleobase sequence of the oligomer sequences shown in any one of tables of sequences or compounds disclosed herein.

In one embodiment, the contiguous nucleobase sequence consists of a total of 15 or 16 nucleobase units, present in, or corresponding to (such as the same base (A,T,C or G) sequence) the nucleobase sequence of the oligomer sequences shown in any one of tables of sequences or compounds disclosed herein.

In one embodiment, the oligomer of the invention may comprise both a polynucleotide region, i.e. a nucleobase region, which typically consists of a contiguous sequence of nucleobases/nucleotides, and a further non-nucleobase region. When referring to the compound of the invention consisting of a nucleobase sequence, the compound may comprise non-nucleobase components, such as a conjugate component.

Alternatively, the oligomer of the invention may consist entirely of a nucleobase region.

In one embodiment, the oligomer according to the invention is not: 5'-GₓGₓcₛaₛaₛgₛcₛaₛtₛcₛcsTₓGₓT-3' or 5'-TₓTₓaₛcₛtₛgₛcₛcₛtₛtₛcₛTₓTₓA-3' or 5'-G_{S}G_{S}c_{S}a₅a_{S}g₅c_{S}a_{S}t_{S}c_{S}c_{S}T_{S}Gₛt-3' or 5'-TₛTₛaₛcₛtₛgₛcₛcₛtₛtₛcₛTₛTₛa-3' (as disclosed in

WO2006/050734) wherein capital letters designate a beta-D-oxy-LNA nucleotide analogue, small letters designate a 2-deoxynucleotide, underline designates either a beta-D-oxy-LNA nucleotide analogue or a 2-deoxynucleotide, subscript "s" designates a phosphorothioate link between neighbouring nucleotides/LNA nucleotide analogues, and subscript "x" designates either a phosphorothioate link or a phosphorodiester link between neighbouring nucleotides/LNA nucleotide analogues.

In one embodiment, the nucteobase sequence of the oligomer according to the invention is not: 5'-GGCAAGCATCCTGT-3' or 5'-TTACTGCCTTCTTA-3'.

In one, not necessarily limiting embodiment, the nucleobase sequence of the oligomer according to the invention is not complementary to a Hif1-alpha target sequence.

In some embodiments of oligomer according to the invention, such as an antisense oligonucleotide which comprises LNA, all LNA C units are 5'methyl-Cytosine (^{m}C).

In most preferred embodiments the oligomer comprises only LNA nucleotide analogues and nucleotides (RNA or DNA, most preferably DNA nucleotides), modified phosphorothioate internucleobase linkages , but with the exception of the 1 or 2 phosphodiester linkages.

### Locked Nucleic Acid (LNA)

The term "LNA" refers to a bicyclic nucleotide analogue, known as "Locked Nucleic Acid". It may refer to an LNA monomer, or, when used in the context of an "LNA oligonucleotide" refers to an oligonucleotide containing one or more such bicyclic nucleotide analogues.

The LNA used in the oligonucleotide compounds of the invention preferably has the structure of the general formula I 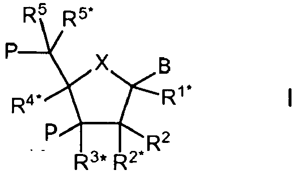 wherein X is selected from -O-, -S-, -N(R^{N*})-, -C(R⁶R^{6*})-;
B is selected from hydrogen, optionally substituted C₁₋₄-alkoxy, optionally substituted C₁₋₄-alkyl, optionally substituted C₁₋₄-acyloxy, natural or non-natural nucleobases, photochemically active groups, thermochemically active groups, chelating groups, reporter groups, and ligands;
P designates the radical position for an internucleoside linkage to a succeeding monomer, or a 5'-terminal group, such internucleoside linkage or 5'-terminal group optionally including the substituent R⁵ or equally applicable the substituent R^{5*};
P* designates an internucleoside linkage to a preceding monomer, or a 3'-terminal group;
R^{4*} and R^{2*} together designate a biradical consisting of 1-4 groups/atoms selected from -C(R^{a}R^{b})-, -C(R^{a})=C(R^{b})-, -C(R^{a})=N-, -O-, -Si(R^{a})₂-, -S-, -SO₂-, - N(R^{a})-, and >C=Z,
wherein Z is selected from O, S, and N(R^{a}), and R^{a} and R^{b} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, cycloalkyl, cycloalkyloxy-carbonyl, cycloalkyloxy, cycloalkylcarbonyl, heterocycloalkyl, heterocycloalkyloxy-carbonyl, heterocycloalkyloxy, heterocycloalkylcarbonyl, amino, mono- and di(C₁₋₆-alkyl)amino, tris(C₁₋₆-alkyl)ammonium, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkylaminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂) or together may form a spiro biradical consisting of a 1-5 carbon atom(s) alkylene chain which is optionally interrupted and/or terminated by one or more heteroatoms/groups selected from -O-, -S-, and -(NR^{N})- where R^{N} is selected from hydrogen and C₁₋₄-alkyl, and
each of the substituents R^{1*}, R², R³, R⁵, R^{5*}, R⁶ and R^{6*}, are independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, cycloalkyl, cycloalkyloxy-carbonyl, cycloalkyloxy, cycloalkylcarbonyl, heterocycloalkyl, heterocycloalkyloxy-carbonyl, heterocycloalkyloxy, heterocycloalkylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkylaminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents together may designate oxo, thioxo, imino, or optionally substituted methylene, or together may form a spiro biradical consisting of a 1-5 carbon atom(s) alkylene chain which is optionally interrupted and/or terminated by one or more heteroatoms/groups selected from -O-, -S-, and -(NR^{N})- where R^{N} is selected
from hydrogen and C₁₋₄-alkyl, and where two adjacent (non-geminal) substituents may designate an additional bond resulting in a double bond; and R^{N*}, when present and not involved in a biradical, is selected from hydrogen and C₁₋₄-alkyl; and basic salts and acid addition salts thereof;

In one embodiment R^{5*} is selected from H, -CH₃, -CH₂-CH₃,- CH₂-O-CH₃, and - CH=CH₂. In one embodiment, R^{4*} and R^{2*} together designate a biradical selected from -C(R^{a}R^{b})-O-, -C(R^{a}R^{b})-C(R^{c}R^{d})-O-, -C(R^{a}R^{b})-C(R^{c}R^{d})-C(R^{e}R^{f})-O-, - C(R^{a}R^{b})-O-C(R^{c}R^{d})-, -C(R^{a}R^{b})-O-C(R^{c}R^{d})-O-, -C(R^{a}R^{b})-C(R^{c}R^{d})-, -C(R^{a}R^{b})-C(R^{c}R^{d})-C(R^{e}R^{f})-, -C(R^{a})=C(R^{b})-C(R^{c}R^{d})-, -C(R^{a}R^{b})-N(R^{c})-, -C(R^{a}R^{b})-C(R^{c}R^{d})- N(R^{e})-, - C(R^{a}R^{b})-N(R^{c})-O-, and -C(R^{a}R^{b})-S-, -C(R^{a}R^{b})-C(R^{c}R^{d})-S-; wherein R^{a}, R^{b}, R^{c}, R^{d}, R^{e}, and R^{f} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂-₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, cycloalkyl, cycloalkyloxy-carbonyl, cycloalkyloxy, cycloalkylcarbonyl, heterocycloalkyl, heterocycloalkyloxy-carbonyl, heterocycloalkyloxy, heterocycloalkylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents together may designate optionally substituted methylene (=CH₂) or together may form a spiro biradical consisting of a 1-5 carbon atom(s) alkylene chain which is optionally interrupted and/or terminated by one or more heteroatoms/groups selected from -O-, -S-, and -(NR^{N})- where R^{N} is selected from hydrogen and C₁₋₄-alkyl.

In a further embodiment R^{4*} and R^{2*} together designate a biradical selected from -CH₂-O-, -CH₂-S-, -CH₂-NH-, -CH₂-N(CH₃)-, -CH₂-CH₂-O-, -CH₂-CH(CH₃)-, -CH₂-CH₂-S-, -CH₂-CH₂-NH-, -CH₂-CH₂-CH₂-, -CH₂-CH₂-CH₂-O-, -CH₂-CH₂-CH(CH₃)-, - CH=CH-CH₂-, -CH₂-O-CH₂-O-, -CH₂-NH-O-, -CH₂-N(CH₃)-O-, -CH₂-O-CH₂-, - CH(CH₃)-O-, -CH(CH₂-O-CH₃)-O-.

All chiral centers may be found in either *R* or *S* orientation.

Preferably, the LNA used in the oligomer of the invention comprises at least one LNA unit according to any of the formulas 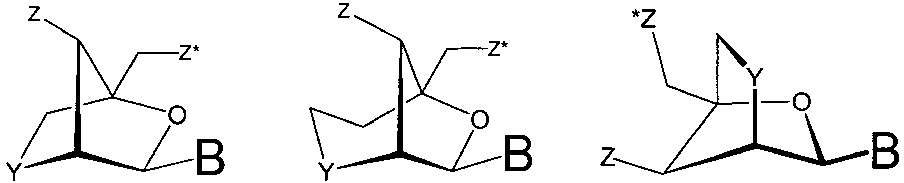 wherein Y is -O-, -S-, -NH-, or N(R^{H}); Z and Z* are independently selected among an internucleoside linkage, a terminal group or a protecting group; B constitutes a natural or non-natural nucleotide base moiety, and R^{H} is selected from hydrogen and C₁₋₄-alkyl.

The term "thio-LNA" comprises a locked nucleotide in which Y in the formulas above represents S. Thio-LNA can be in both beta-D and alpha-L-configuration.

The term "amino-LNA" comprises a locked nucleotide in which Y in the formulas above is selected from -N(H)-, and N(R^{H})-, where R^{H} is selected from hydrogen and C₁₋₄-alkyl. Amino-LNA can be in both beta-D and alpha-L-configuration.

The term "oxy-LNA" comprises a locked nucleotide in which Y in the formulas above represents -O-. Oxy-LNA can be in both beta-D and alpha-L-configuration.

The term "ENA" comprises a locked nucleotide in which Y in the formulas above is -CH₂-O- (where the oxygen atom of -CH₂-O- is attached to the 2'-position relative to the base B). ENA can be in both beta-D and alpha-L-configuration. In a preferred embodiment LNA is selected from beta-D-oxy-LNA, alpha-L-oxy-LNA, beta-D-amino-LNA and beta-D-thio-LNA, in particular beta-D-oxy-LNA. Specifically preferred LNA units are shown in scheme 2:

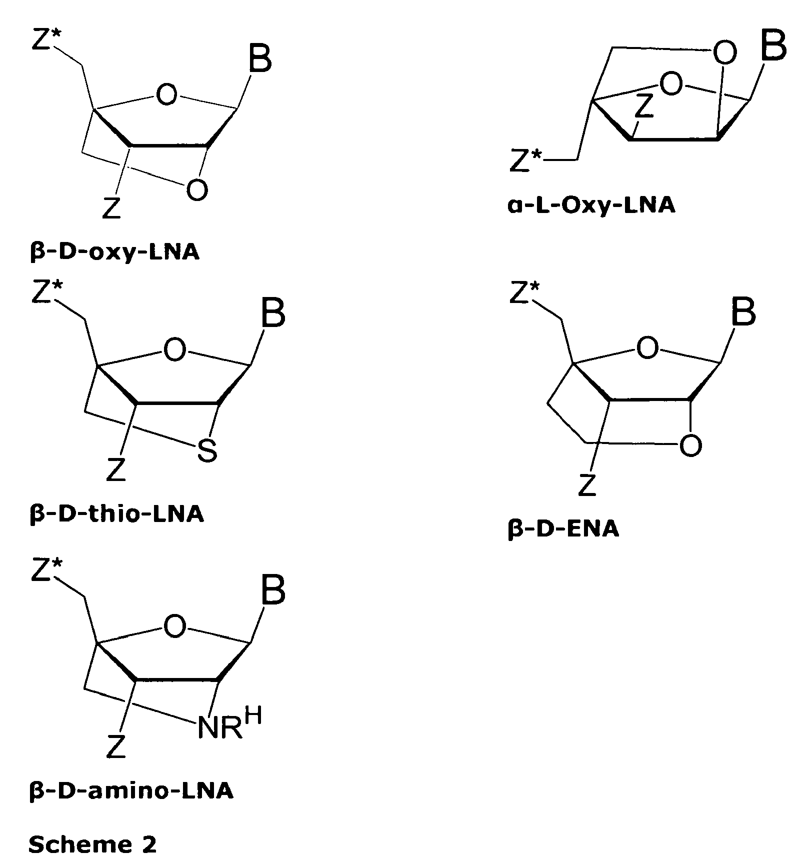

Preferably, the LNA used in the oligomer of the invention comprises internucleoside linkages selected from -O-P(O)₂-O-, -O-P(O,S)-O-, -O-P(S)₂-O-, -S-P(O)₂-O-, -S-P(O,S)-O-, -S-P(S)₂-O-, -O-P(O)₂-S-, -O-P(O,S)-S-, -S-P(O)₂-S-, -O-PO(R^{H})-O-, O-PO(OCH₃)-O-, -O-PO(NR^{H})-O-, -O-PO(OCH₂CH₂S-R)-O-, -O-PO(BH₃)-O-, -O-PO(NHR^{H})-O-, -O-P(O)₂-NR^{H}-, -NR^{H}-P(O)₂-O-, -NR^{H}-CO-O-, where R^{H} is selected form hydrogen and C₁₋₄-alkyl.

When used herein, the term "DNA intercalator" means a group which can intercalate into a DNA or RNA helix, duplex or triplex. (Examples of functional parts of DNA intercalators are acridines, anthracene, quinones such as anthraquinone, indole, quinoline, isoquinoline, dihydroquinones, anthracyclines, tetracyclines, methylene blue, anthracyclinone, psoralens, coumarins, ethidium-halides, dynemicin, metal complexes such as 1,10-phenanthroline-copper, tris(4,7-diphenyl-1,10-phenanthroline)ruthenium-cobalt-enediynes such as calcheamicin, porphyrins, distamycin, netropcin, viologen, daunomycin.)? Especially interesting examples are acridines, quinones such as anthraquinone, methylene blue, psoralens, coumarins, and ethidium-halides.

In the present context, the term "photochemically active groups" covers compounds which are able to undergo chemical reactions upon irradiation with light. Illustrative examples of functional groups hereof are quinones, especially 6-methyl-1,4-naphtoquinone, anthraquinone, naphthoquinone, and 1,4-dimethyl-anthraquinone, diazirines, aromatic azides, benzophenones, psoralens, diazo compounds, and diazirino compounds.

In the present context "thermochemically reactive group" is defined as a functional group which is able to undergo thermochemically-induced covalent bond formation with other groups. Illustrative examples of functional parts thermochemically reactive groups are carboxylic acids, carboxylic acid esters such as activated esters, carboxylic acid halides such as acid fluorides, acid chlorides, acid bromide, and acid iodides, carboxylic acid azides, carboxylic acid hydrazides, sulfonic acids, sulfonic acid esters, sulfonic acid halides, semicarbazides, thiosemicarbazides, aldehydes, ketones, primary alcohols, secondary alcohols, tertiary alcohols, phenols, alkyl halides, thiols, disulphides, primary amines, secondary amines, tertiary amines, hydrazines, epoxides, maleimides, and boronic acid derivatives.

In the present context, the term "chelating group" means a molecule that comprises more than one binding site and frequently binds to another molecule, atom or ion through more than one binding site at the same time. Examples of functional parts of chelating groups are iminodiacetic acid, nitrilotriacetic acid, ethylenediamine tetraacetic acid (EDTA), aminophosphonic acid, etc.

In the present context, the term "reporter group" means a group which is detectable either by itself or as a part of an detection series. Examples of functional parts of reporter groups are biotin, digoxigenin, fluorescent groups (groups which are able to absorb electromagnetic radiation, *e*.*g*. light or X-rays, of a certain wavelength, and which subsequently reemits the energy absorbed as radiation of longer wavelength; illustrative examples are dansyl (5-dimethylamino)-1-naphthalenesulfonyl), DOXYL (*N*-oxyl-4,4-dimethyloxazolidine), PROXYL (*N*-oxyl-2,2,5,5-tetramethylpyrrolidine), TEMPO (*N*-oxyl-2,2,6,6-tetramethylpiperidine), dinitrophenyl, acridines, coumarins, Cy3 and Cy5 (trademarks for Biological Detection Systems, Inc.), erytrosine, coumaric acid, umbelliferone, Texas Red, rhodamine, tetramethyl rhodamine, Rox, 7-nitrobenzo-2-oxa-1-diazole (NBD), pyrene, fluorescein, europium, ruthenium, samarium, and other rare earth metals), radioisotopic labels, chemiluminescence labels (labels that are detectable via the emission of light during a chemical reaction), spin labels (a free radical (*e*.*g*. substituted organic nitroxides) or other paramagnetic probes (*e*.*g*. Cu²⁺, Mg²⁺) bound to a biological molecule being detectable by the use of electron spin resonance spectroscopy), enzymes (such as peroxidases, alkaline phosphatases, β-galactosidases, and glucose oxidases), antigens, antibodies, haptens (groups which are able to combine with an antibody, but which cannot initiate an immune response by itself, such as peptides and steroid hormones), carrier systems for cell membrane penetration such as: fatty acid residues, steroid moieties (cholesterol), vitamin A, vitamin D, vitamin E, folic acid peptides for specific receptors, groups for mediating endocytose, epidermal growth factor (EGF), bradykinin, and platelet derived growth factor (PDGF). Especially interesting examples are biotin, fluorescein, Texas Red, rhodamine, dinitrophenyl, digoxigenin, ruthenium, europium, Cy5 and Cy3.

In the present context "ligand" means something which binds. Ligands can comprise functional groups such as: aromatic groups (such as benzene, pyridine, naphtalene, anthracene, and phenanthrene), heteroaromatic groups (such as thiophene, furan, tetrahydrofuran, pyridine, dioxane, and pyrimidine), carboxylic acids, carboxylic acid esters, carboxylic acid halides, carboxylic acid azides, carboxylic acid hydrazides, sulfonic acids, sulfonic acid esters, sulfonic acid halides, semicarbazides, thiosemicarbazides, aldehydes, ketones, primary alcohols, secondary alcohols, tertiary alcohols, phenols, alkyl halides, thiols, disulphides, primary amines, secondary amines, tertiary amines, hydrazines, epoxides, maleimides, C₁-C₂₀ alkyl groups optionally interrupted or terminated with one or more heteroatoms such as oxygen atoms, nitrogen atoms, and/or sulphur atoms, optionally comprising aromatic or mono/polyunsaturated hydrocarbons, polyoxyethylene such as polyethylene glycol, oligo/polyamides such as poly-β-alanine, polyglycine, polylysine, peptides, oligo/polysaccharides, oligo/polyphosphates, toxins, antibiotics, cell poisons, and steroids, and also "affinity ligands", *i*.*e*. functional groups or biomolecules that have a specific affinity for sites on particular proteins, antibodies, poly- and oligosaccharides, and other biomolecules.

The LNA used in the oligonucleotide compounds of the invention preferably has the structure of the general formula 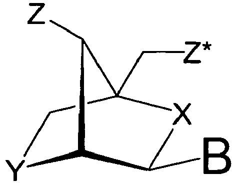
where X and Y are independently selected among the groups -O-,
-S-, -N(H)-, N(R)-, -CH₂- or -CH- (if part of a double bond),
-CH₂-O-, -CH₂-S-, -CH₂-N(H)-, -CH₂-N(R)-, -CH₂-CH₂- or -CH₂-CH- (if part of a double bond), -CH=CH-, where R is selected from hydrogen and C₁₋₄-alkyl; Z and Z* are independently selected among an internucleoside linkage, a terminal group or a protecting group; B constitutes a natural or non-natural nucleotide base moiety; and the asymmetric groups may be found in either orientation.

Preferably the LNA units comprise at least one beta-D-oxy-LNA unit(s) such as 2, 3, 4, 5 or 6 beta-D-oxy-LNA units.

In the present context, the term "C1-4-alkyl" is intended to mean a linear or branched saturated hydrocarbon chain wherein the chain has from one to four carbon atoms, such as methyl, ethyl, n-propyl, isopropyl, n-butyl, isobutyl, sec-butyl and tert-butyl.

The oligomer of the invention, such as the antisense oligonucleotide, may comprise more than one type of LNA unit. Suitably, the compound may comprise both beta-D-oxy-LNA, and one or more of the following LNA units: thio-LNA, amino-LNA, oxy-LNA, ena-LNA and/or alpha-LNA in either the D-beta or L-alpha configurations or combinations thereof.

Benefits of utilising LNA, and methods of preparing and purifying LNA and LNA oligonucleotides are disclosed in PCT/DK2006/000512.

In one embodiment, the oligomer of the invention does not comprise any RNA units.

In one embodiment, at least one of the nucleobases present in the oligomer is a modified nucleobase selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

### RNAse H recruitment and Gapmer oligonucleotides.

The oligomer of the invention is a gapmer of formula A-B-C, or optionally A-B-C-D or D-A-B-C.

Region A may consist of 1, 2, 3, 4, 5 or 6 contiguous LNA units; region B may consist of 6,7, 8, 9, 10, 11 or 12 nucleotide units, such as DNA units, or a combination of DNA and other units capable of recruiting RNAseH, such as alpha-L LNA units.

In one embodiment, the oligomer comprises 2, 3, 4, 5 or 6 LNA units (i.e. the total of LNA units in regions A and C. In one embodiment at least one of region A and/or C consists of 2 LNA units. In one embodiment, region A and C both consist of 2 LNA units. In one embodiment A consists of 1 LNA unit. In one embodiment A consists of 2 LNA units. In one embodiment A consists of 3 LNA units. In one embodiment C consists of 1 LNA unit. In one embodiment C consists of 2 LNA units. In one embodiment C consists of 3 LNA units. In one embodiment B consists of 7 nucleobase units. In one embodiment B consists of 8 nucleobase units. In one embodiment B consists of 9 nucleobase units. In one embodiment B consists of 10 nucleobase units. In one embodiment A and/or C consists of 4 or 5 nucleobase units. In one embodiment B comprises of between 1-9 DNA units, such as 2, 3, 4, 5, 6, 7 or 8 DNA units. In one embodiment B consists of only DNA units - i.e. 7, 8 or ) DNA units (2'deoxyribonucleoside). In one embodiment B comprises of at least one LNA unit which is in the alpha-L configuration, such as 2, 3, 4, 5, 6, 7, 8 or 9 LNA units in the alpha-L-configuration. In one embodiment B comprises of at least one alpha-L-oxy LNA unit or wherein all the LNA units in the alpha-L- configuration are alpha-L-oxy LNA units. In one embodiment the number of nucleobases in A-B-C are selected from the group consisting of: 1-8-1, 1-8-2, 2-8-1, 2-8-2, 3-8-3, 2-8-3, and 3-8-2. In one embodiment the number of nucleobases in A-B-C are selected from the group consisting of: 3-9-3, 3-10-3, 4-8-3, or 3-8-4. In one embodiment the number of nucleobases in A-B-C are selected from the group consisting of: 1-9-1, 1-9-2, 2-9-1, 2-9-2, 3-9-2, and 2-9-3. In one embodiment the number of nucleobase in A-B-C are selected from the group consisting of: 2-7-1, 1-7-2, 2-7-2, 3-7-3, 2-7-3, 3-7-2, 3-7-4, and 4-7-3. In one embodiment A and C both consist of two LNA units each, and B consists of 8 nucleobase units, preferably DNA units. In one embodiment the LNA units of A and C are independently selected from oxy-LNA, thio-LNA, and amino-LNA, in either of the beta-D and alpha-L configurations or combinations thereof. In one embodiment LNA units of A and C are beta-D-oxy-LNA. In one embodiment A consists of 2 LNA units neither of which are alpha-L-oxy LNA. In one embodiment C consists of 2 LNA units neither of which are alpha-L-oxy LNA. In one embodiment A does not comprise any thio-LNA nucleobases. In one embodiment C does not comprise any thio-LNA nucleobases. In one embodiment A does not comprise any amino-LNA nucleobases. In one embodiment C does not comprise any amino-LNA nucleobases. When present, D typically consists of a single DNA unit. In one embodiment, there is no region D. In one embodiment regions A and C consist of either 1 or 2 nucleobase units. In one embodiment, region C consists of 2 residues, and region A consists of 1, 2 or 3 nucleobase units. In one embodiment, region A consists of 2 residues, and region C consists of 1, 2 or 3 nucleobase units.

In one embodiment the 3' and/or 5' residues of region B are an LNA nucleobase in the alpha L configurations, such as alpha-L-oxy LNA. In one embodiment, which may be the same of different, at least one of the nucleobase units other than the 3' and/or 5' residues of region B are an LNA nucleobase in the alpha L configurations, such as alpha-L-oxy LNA.

Region B is essential in terms of recruitment of RNAseH - it is therefore highly preferred that region B comprises a nucleobase sequence that is capable of recruiting RNAseH. DNA nucleotides are highly preferred, although as disclosed in WO2004/046160, a DNA unit (or more) in region B may be substituted with one (or more) LNA units which are in the alpha-L configuration, particularly preferred being alpha-L-oxy LNA. Suitably 1, 2, 3, 4, 5, 6, 7, 8 or 9 of the DNA units of region B may be substituted in this manner, although when such substitutions are made it is recommended that, for example only 1, 2, or 3 of such substitutions are made in region B.

EP 1 222 309 provides *in vitro* methods for determining RNaseH activity, which may be used to determine the ability to recruit RNaseH. A oligomer is deemed capable of recruiting RNase H if, when provided with the complementary RNA target, it has an initial rate, as measured in pmol/I/min, of at least 1 %, such as at least 5%, such as at least 10% or less than 20% of the equivalent DNA only oligonucleotide, with no 2' substitutions, with phosphorothioate linkage groups between all nucleotides in the oligonucleotide, using the methodology provided by Example 91 - 95 of EP 1 222 309.

As referred to above, one or more of the DNA nucleotides in the central domain (B) may be substituted with one or more nucleotide analogues which are capable of recruiting RNAse H, or even all the DNA nucleotides may be substituted with nucleotide analogues which are capable or recruiting RNAse H. LNA nucleobases which form the alpha-L configuration, such as alpha-L-oxy LNA are particularly preferred nucleotide analogues which may be incorporated into region B as they are capable of recruiting RNAseH. In this respect region B may comprise both alpha - L- LNA and DNA units. Region B may comprise an alpha - L- LNA unit, which may, for example, be at position 1, 2, 3, 4, 5, 6, 7, 8, or 9 of region B (as determined from either the 3' or 5' end), and in one embodiment the remaining nucleobases of region B may be DNA, or alternatively region B may comprise one or more further alpha-L- LNA units, such as 2, 3, 4, 5, 6, 7, 8, or 9 further alpha-L-LNA units. In one embodiment, region B comprises 2 alpha-L-LNA units, and the remaining nucleobase units are DNA. In a further embodiment, region B comprises 3 alpha-L-LNA units, and the remaining nucleobase units are DNA. The alpha-L- units may, in one embodiment be positioned at the 5' and or 3' positions of region B, and/or in a non terminal position of region B. Where more than one alpha-L-LNA unit is present in region B, region B may comprise a sequence where the alpha-LNA units are either adjacent to each other (i.e. ant least 5' -LNA-LNA- 3') and/or where the alpha LNA units are non-adjacent, i.e. separated by at least 1, such as 1, 2, 3, 4, 5, 6, 7, 8, 9 or 10 alternative nucleobases or nucleotides, such as DNA units.

In one embodiment, the gapmer, of formula A-B-C, further comprises a further region, D, which consists or comprises, preferably consists, of one DNA sugar residue terminal of the 3' region (C) of the oligomeric compound. Alternatively, region D may be immediately 5' (i.e. adjacent to) to region A.

Preferably the LNA units of the oligomer, such as an antisense oligonucleotide, of the invention are selected from one or more of the following: thio-LNA, amino-LNA, oxy-LNA, ena-LNA and/or alpha-LNA in either the D-beta or L-alpha configurations or combinations thereof. Beta-D-oxy-LNA is a preferred LNA for use in the oligomer of the invention, particularly in regions A and C (where as alpha-L-LNA are preferred, when present, in region B). Thio-LNA may also be preferred for use in the oligomer of the invention. Amino-LNA may also be preferred for use in the oligomer of the invention. Oxy-LNA may also be preferred for use in the oligomer of the invention. Ena-LNA may also be preferred for use in the oligomer of the invention. Alpha-LNA may also be preferred for use in the oligomer of the invention.

### Internucleobase/Internucleoside Linkages

The terms "linkage group" or "Internucleoside linkage" or "Internucleobase linkage" are intended to mean a group capable of covalently coupling together two nucleotides, two nucleotide analogues, and a nucleotide and a nucleotide analogue, etc. Specific and preferred examples include phosphate groups and phosphorothioate groups.

Internucleoside linkages include those listed within PCT/DK2006/000512, for example the internucleoside linkages listed on the first paragraph of page 34 of PCT/DK2006/000512.

Phosphorothioate internucleotide linkages are used for the gap region (B). Other than the phosphodiester linkages Phosphorothioate linkages may also be used for the flanking regions (A and C, and for linking C to D, and D). B

The internucleobase linkages in the gap region B of the oligomer phosphorothioate are so as to allow RNase H cleavage of targeted RNA.

In one embodiment, the internucleoside linkages adjacent to or between DNA units are phosphorothioate linkages. In one embodiment, the linkages between at least one pair, such as two (independent or consecutive)pairs, of consecutive LNA units, such as 2 LNA units in region A or C, is a phosphodiester linkage. In one embodiment, the linkages between consecutive LNA units such as 2 LNA units in region A and/or C, are phosphodiester linkages. All the remaining internucleoside linkages, or the remaining internucleoside linkages, are phosphorothioate linkages. In some embodiments region A comprises at least one phosphodiester linkage between two nucleotide analogue units, or a nucleotide analogue unit and a nucleobase unit of Region B. In some embodiments region C comprises at least one phosphodiester linkage between two nucleotide analogue units, or a nucleotide analogue unit and a nucleobase unit of Region B. In some embodiments, region C comprises at least one phosphodiester linkage between a nucleotide analogue unit and a nucleobase unit of Region D. In some embodiments, region A comprises at least one phosphodiester linkage between a nucleotide analogue unit and a nucleobase unit of region D. In some embodiments the linkage between the 3' nucleotide analogue of region A and the 5' nucleobase of region B is a phosphodiester. In some embodiments the linkage between the 3' nucleobase of region B and the 5' nucleotide analogue of region C is a phosphodiester. In some embodiments the linkage between the two adjacent nucleotide analogues at the 5' end of region A are phosphodiester. In some embodiments the linkage between the two adjacent nucleotide analogues at the 3' end of region C is phosphodiester. In some embodiments the linkage between the two adjacent nucleotide analogues at the 3' end of region A is phosphodiester. In some embodiments the linkage between the two adjacent nucleotide analogues at the 5' end of region C is phosphodiester. In some embodiments all the linkage s between nucleotide analogues present in the compound of the invention are phosphodiester. In some embodiments, such as the embodiments referred to above, where suitable and not specifically indicated, all remaining linkage s are either phosphorothioate. In one embodiment the only phosphodiester linkages are found within regions A and/or C, or between regions A-B and/or C-B.

When referring to specific gapmer oligonucleotide sequences, such as those provided herein, when the C residues are annotated as 5'methyl modified cytosine, in one embodiment, one or more of the Cs present in the oligonucleotide may be unmodified C residues.

### Phosphodiester/Phosphorothioate Chimeras

The present inventors have found that the introduction of a single phosphodiester into a gapmer can enhance the bioavailability of the oligomer *in vivo*. Furthermore, the present inventors have found that the introduction of a single phosphodiester into a gapmer can result in an enhanced accumulation of the oligomer in kidney cells, particularly kidney cortex cells. Suitably, the enhanced distribution to the kidney cells may be seen when comparing the relative amounts which accumulate in the liver and the kidney. Suitably, as described herein, one further phosphodiester linkages may be used.

The use of oligonucleotides of between 10 - 16 nucleobases in length, such as shortmers, is, in many instances, advantageous as they have enhanced *in vivo* bioavailability due to their small size. However, their small size also results in an increased risk of excretion in the kidney. The present inventors were therefore surprised to find that the introduction of a single phosphodiester linkage, or two phosphodieater linkages, in an otherwise phosphorothioate oligomer shortmer was sufficient to enhance the bioavailability and the accumulation rather than the excretion from the kidney. The phosphodiester linkages should preferably be placed (inserted) either between two adjacent nucleotide analogues, such as LNA, present in regions A and or C, and/or, in one embodiment, between an adjacent nucleotide analogue, such as LNA, present in regions A and or C, and a nucleobase of region B.

The present invention therefore also provides for a method for modifying a fully phosphorothioate oligomer to enhance its accumulation or bioavailability in an organ, such as the kidney said method comprising replacing a total of one or two of the phosphorothioate linkages with phosphodiester bonds.

It will be understood that by the term modifying, we refer to both a method of manufacture of oligomer and/or a method of designing. In this regards it is not considered that one should take a physical fully phosphorothioate oligomer and replace one or two of the phosphorothioate linkages with phosphodiester linkages, but, instead it is a method of designing modified phosphorothioate oligomers, by replacing one or two of the phosphorothioate linkages with phosphodiester linkages. Subsequently the oligomers can be made using routine oligonucleotide synthesis chemistry.

Whilst the present inventors consider that such modified oligomers are particularly useful for shortmer oligonucleotides, it is considered that the method is not limited to oligonucleotides of, say between 10-14 nucleobases, but is also applicable to longer oligomers, such as oligomers consisting of a contiguous nucleobase sequence of a total of 10 - 20 nucleobase units, such as 11, 12, 13, 14, 15, 16, 17, 18, 19, or 20 nucleobase units.

### Conjugates

In the present context the term "conjugate" is intended to indicate a heterogenous molecule formed by the covalent attachment of a compound as described herein (i.e. a compound comprising a sequence of nucleotides analogues) to one or more non-nucleotide/non- nucleotide-analogue, or non-polynucleotide moieties. Examples of non-nucleotide or non- polynucleotide moieties include macromolecular agents such as proteins, fatty acid chains, sugar residues, glycoproteins, polymers, or combinations thereof. Typically proteins may be antibodies for a target protein. Typical polymers may be polyethylene glycol. When the compound of the invention consists of a nucleobase sequence, it may, in one embodiment further comprise a non-nucleobase portion, such as the above conjugates.

The invention also provides for a conjugate comprising the compound of the invention and at least one non-nucleotide or non-polynucleotide moiety covalently attached to said compound. PCT/DK2006/000512 provides suitable ligands and conjugates.

In one embodiment of the invention, the oligonucleotide may be linked to ligands/conjugates, which may be used, *e*.*g*. to increase the cellular uptake of the oligonucleotide. This conjugation can take place at the terminal positions 5'/3'-OH but the ligands may also take place at the sugars and/or the bases. The 3' -OH is preferred site for cholesterol conjugation.

In a preferred embodiment, the oligonucleotide of the invention is conjugated with a moiety which improvise the in vivo uptake, such as cholesterol.

Thus, the oligomeric compound may, e.g., be conjugated or form chimera with non-nucleotide or non-polynucleotide moieties including Peptide Nucleic Acids (PNA), proteins (e.g. antibodies for a target protein), macromolecules, low molecular weight drug substances, fatty acid chains, sugar residues, glycoproteins, polymers (e.g. polyethylene glycol), micelle-forming groups, antibodies, carbohydrates, receptor-binding groups, steroids such as cholesterol, polypeptides, intercalating agents such as an acridine derivative, a long-chain alcohol, a dendrimer, a phospholipid and other lipophilic groups or combinations thereof, etc., just as the Oligomeric compound may be arranged in dimeric or dendritic structures.

In one embodiment referring to the conjugate, the non-nucleotide or non-polynucleotide moiety consists or comprise a sterol group such as cholesterol.

Other such non-nucleotide or non-polynucleotide moieties include, but are not limited to, lipid moieties such as a cholesterol moiety, cholic acid, a thioether, e.g. Hexyl-s-tritylthiol, a thiocholesterol, an aliphatic chain, e.g., dodecandiol or undecyl residues, a phospholipids, e.g., di-hexadecyl-rac-glycerol or triethylammonium 1,2-di-o-hexadecyl-rac-glycero-3-h-phosphonate, a polyamine or a polyethylene glycol chain, an adamantane acetic acid, a palmityl moiety, an octadecylamine or hexylamino-carbonyl-oxycholesterol moiety.

The oligomers of the invention may also be conjugated to active drug substances, for example, aspirin, ibuprofen, a sulfa drug, an antidiabetic, an antibacterial or an antibiotic.

In one embodiment of the invention the oligomeric compound is linked to ligands/conjugates, which may be used, *e*.*g*. to increase the cellular uptake of antisense oligonucleotides. PCT/DK2006/000512 provides suitable ligands and conjugates.

The invention also provides for a conjugate comprising the compound according to the invention as herein described, and at least one non-nucleotide or non-polynucleotide moiety covalently attached to said compound. Therefore, in one embodiment where the compound of the invention consists of a specified nucleic acid, as herein disclosed, the compound may also comprise at least one non-nucleotide or non-polynucleotide moiety (e.g. not comprising one or more nucleotides or nucleotide analogues) covalently attached to said compound.

### Applications

The oligomers of the invention may be utilized as research reagents for, for example, diagnostics, therapeutics and prophylaxis.

In research, such oligomers may be used to specifically inhibit the synthesis of the target protein (typically by degrading or inhibiting the mRNA and thereby prevent protein formation) in cells and experimental animals thereby facilitating functional analysis of the target or an appraisal of its usefulness as a target for therapeutic intervention.

In diagnostics the oligomers may be used to detect and quantitate the target expression in cell and tissues by Northern blotting, in-situ hybridisation or similar techniques.

For therapeutics, an animal or a human, suspected of having a disease or disorder, which can be treated by modulating the expression of the target - is treated by administering antisense compounds (oligomers) in accordance with this invention. Further provided are methods of treating an animal, such as a mouse or rat, or preferably, treating a human, suspected of having or being prone to a disease or condition, associated with expression of the target by administering a therapeutically or prophylactically effective amount of one or more of the oligomers or compositions of the invention.

The pharmaceutical composition according to the invention may be used for the treatment of conditions associated with:

ApoB-100 targeting oligomers - abnormal levels of apoB-100, atherosclerosis, hypercholesterolemia or hyperlipidemia. It will be recognised that the ApoB-100 targeting oligomers may be combined with further therapeutic agents in the pharmaceutical composition according to the invention - such as those further therapeutic agents provided in US 60/896,419 and/or WO2007/031081.

Hif-1alpha targeting oligomers - abnormal levels of Hif-1alpha - artherosclerosis, psoriasis, diabetic retinopathy, macular degeneration, rheumatoid arthritis, asthma, inflammatory bowel disease, warts, allergic dermatitis, inflammation, and skin inflammation. It will be recognised that the Hif-1alpha targeting oligomers may be combined with further therapeutic agents in the pharmaceutical composition according to the invention - such as those further therapeutic agents provided in WO2006/050734.

Suitable dosages, formulations, administration routes, compositions, dosage forms, combinations with other therapeutic agents, pro-drug formulations are also provided in PCT/DK2006/000512.

The invention also provides for a pharmaceutical composition comprising a compound or a conjugate as herein described or a conjugate, and a pharmaceutically acceptable diluent, carrier or adjuvant. PCT/DK2006/000512 provides suitable and preferred pharmaceutically acceptable diluent, carrier and adjuvants.

### Pharmaceutical application

The pharmaceutical application of LNA oligomers is, for example, described in WO2007/031081 (anti-ApoB oligomers) and WO2006/050734 (anti-hif1-alpha oligomers).

### Embodiments

The following further embodiments may be combined with the features of the invention as referred to herein:
1. An oligomer according to claim 1 consisting of a contiguous nucleobase sequence of a total of 10, 11, 12, 13 or 14 nucleobase units, wherein the contiguous nucleobase sequence is of formula (5' - 3'), A-B-C, wherein: A consists of 1, 2 or 3 LNA units; B consists of 7, 8 or 9 contiguous nucleobase units which are capable of recruiting RNAseH when formed in a duplex with a complementary RNA molecule (such as a mRNA target); and C consists of 1, 2 or 3 LNA units.
2. The oligomer according to embodiment 1, wherein A consists of 1 LNA unit.
3. The oligomer according to embodiment 1, wherein A consists of 2 LNA units.
4. The oligomer according to embodiment 1, wherein A consists of 3 LNA units.
5. The oligomer according to any one of embodiments 1-4, wherein C consists of 1 LNA unit.
6. The oligomer according to any one of embodiments 1-4, wherein C consists of 2 LNA units.
7. The oligomer according to any one of embodiments 1-5, wherein C consists of 3 LNA units.
8. The oligomer according to any one of embodiments 1-7, wherein B consists of 7 nucleobase units.
9. The oligomer according to any one of embodiments 1-7, wherein B consists of 8 nucleobase units.
10.The oligomer according to any one of embodiments 1-7, wherein B consists of 9 nucleobase units.
11.The oligomer according to any one of embodiments 1 - 10, wherein B comprises of between 1-9 DNA units, such as 2, 3, 4, 5, 6, 7 or 8 DNA units..
12.The oligomer according to embodiment 11, wherein B consists of DNA units.
13.The oligomer according to any one of embodiments 1-11, wherein B comprises of at least one LNA unit which is in the alpha-L configuration, such as 2, 3, 4, 5, 6, 7, 8 or 9 LNA units in the alpha-L-configuration.
14.The oligomer according to embodiment 13, wherein B comprises of at least one alpha-L-oxy LNA unit or wherein all the LNA units in the alpha-L-configuration are alpha-L-oxy LNA units.
15.The oligomer according to any one of embodiments 1 - 14, wherein the number of nucleobases in A-B-C are selected from the group consisting of: 1-8-2, 2-8-1, 2-8-2, 3-8-3, 2-8-3, 3-8-2.
16.The oligomer according to any one of embodiments 1- 15, wherein both A and C both consist of two LNA units each, and B consists of 8 nucleobase units, preferably DNA units.
17.The oligomer according to any one of embodiments 1 - 16, wherein the LNA units of A and C are independently selected from oxy-LNA, thio-LNA, and amino-LNA, in either of the beta-D and alpha-L configurations or combinations thereof.
18.The oligomer according to embodiment 17, wherein the LNA units of A and C are beta-D-oxy-LNA.
19.The oligomer according to any one of embodiments 1 - 18, wherein A consists of 2 LNA units neither of which are alpha-L-oxy LNA.
20.The oligomer according to any one of embodiments 1 - 19, wherein C consists of 2 LNA units neither of which are alpha-L-oxy LNA.
21.The oligomer according to any one of embodiments 1 - 20, wherein A does not comprise any thio-LNA nucleobases.
22.The oligomer according to any one of embodiments 1 - 21, wherein C does not comprise any thio-LNA nucleobases.
23.The oligomer according to any one of embodiments 1 - 22, wherein A does not comprise any amino-LNA nucleobases.
24.The oligomer according to any one of embodiments 1-23 wherein C does not comprise any amino-LNA nucleobases.
25. The oligomer according to any one of embodiments 1 - 24, wherein the contiguous nucleobase sequence is complementary to a corresponding region of a mammalian, such as a human mRNA, selected form the group consisting of ApoB-100, PCSK9, Hif1alpha, FABP4 and profilin.
26. The oligomer according to any one of embodiments 1 - 25, wherein the contiguous nucleobase sequence is selected from the group consisting of a contiguous nucleobase seqeunce present in, or corresponding to a nucleobase sequence of the oligomer sequences shown in any one of tables 1, 2, 3, 4, 5, 6, 7, 8, 9, 10 or 11.
27. A conjugate comprising the oligomer according to any one of the embodiments 1-26 and at least one non-nucleotide or non-polynucleotide moiety covalently attached to said compound.
28. A pharmaceutical composition comprising an oligomer as defined in any of embodiments 1-26 or a conjugate as defined in embodiment 33, and a pharmaceutically acceptable diluent, carrier, salt or adjuvant.
29. A pharmaceutical composition according to 28, wherein the oligomer is constituted as a pro-drug.
30. The in vitro use of an oligomer according to any one of embodiments 1 - 36 for the reduction in cellular concentration of a mRNA in a mammalian cell.
37.The use of an oligomer according to embodiment 30, wherein the mRNA is a human mRNA selected form the group consisting of ApoB-100, PCSK9, Hif1alpha, FABP4 and profilin.
38. An in vitro method for the reduction in the cellular concentration of a mRNA in a mammalian cell, said method comprising the administration of an oligomer according to any one of embodiments 1 - 26 to the mammalian cell, wherein said mammalian cell comprises an mRNA species which comprises a nucleobase sequence which is complementary to said oligomer.

### EXAMPLES

### Example 1: Monomer synthesis

The LNA nucleotide analogue building blocks (e.g. β-D-oxy-LNA, β-D-thio-LNA, β-D-amino-LNA and α-L-oxy-LNA) can be prepared following established published procedures - for example see WO2007/031081.

### Example 2: Oligonucleotide synthesis

Oligonucleotides were synthesized according to the method described and referenced in WO07/031081.

### Oligonucleotide compounds

In SEQ ID NOS: 16-40, upper case letters indicates nucleotide analogue units and subscript "s" represents phosphorothiote linkage. Absence of "s" indicates phosphodiester linkage.

| **Test substance** | **Sequence** | **Size** | |
|---|---|---|---|
| SEQ ID NO: 1 | 5'-CAGC ATTG GTAT TCAG-3' | 16 | Antisense motif |
| SEQ ID NO: 2 | 5'-CAGC ATTG GTAT TCA-3' | 15 | Antisense motif |
| SEQ ID NO: 3 | 5'-AGCA TTGG TATT CAG-3' | 15 | Antisense motif |
| SEQ ID NO: 4 | 5'-CAGC ATTG GTAT TC-3' | 14 | Antisense motif |
| SEQ ID NO: 5 | 5'-AGCA TTGG TATT CA-3' | 14 | Antisense motif |
| SEQ ID NO: 6 | 5'-GCAT TGGT ATTC AG-3' | 14 | Antisense motif |
| SEQ ID NO: 7 | 5'-CAGC ATTG GTAT T-3' | 13 | Antisense motif |
| SEQ ID NO: 8 | 5'-AGCA TTGG TATT C-3' | 13 | Antisense motif |
| SEQ ID NO: 9 | 5'-GCAT TGGT ATTC A-3' | 13 | Antisense motif |
| SEQ ID NO: 10 | 5'-CATT GGTA TTCA G-3' | 13 | Antisense motif |
| SEQ ID NO: 11 | 5'-CAGC ATTG GTAT-3' | 12 | Antisense motif |
| SEQ ID NO: 12 | 5'-AGCA TTGG TATT-3' | 12 | Antisense motif |
| SEQ ID NO: 13 | 5'-GCAT TGGT ATTC-3' | 12 | Antisense motif |
| SEQ ID NO: 14 | 5'-CATT GGTA TTCA-3' | 12 | Antisense motif |
| SEQ ID NO: 15 | 5'-ATTG GTAT TCAG-3' | 12 | Antisense motif |
| SEQ ID NO: 16 | 5'-**AₛGₛ^{Me}Cₛ**aₛtₛtₛgₛgₛtₛaₛtₛ**Tₛ^{Me}CₛAₛ**g-3' | 16 | Motif #1 |
| SEQ ID NO: 17 | 5'-**AₛGₛ^{Me}Cₛ**aₛtₛtₛgₛgₛtₛaₛtₛ**Tₛ^{Me}CₛA**-3' | 14 | Motif #5 |
| SEQ ID NO: 18 | 5'**-AGₛ^{Me}Cₛ**aₛtₛtₛgₛgₛtₛaₛtₛ**Tₛ^{Me}CₛA**-3' | 14 | Motif #5 |
| SEQ ID NO: 19 | 5'**-AₛG^{Me}Cₛ**aₛtₛtₛgₛgₛtₛaₛtₛ**Tₛ^{Me}CₛA-**3' | 14 | Motif #5 |
| SEQ ID NO: 20 | 5'-**AₛGₛ^{Me}C**aₛtₛtₛgₛgₛtₛaₛtₛ**Tₛ^{Me}CₛA**-3' | 14 | Motif #5 |
| SEQ ID NO: 21 | 5'-**AₛGₛ^{Me}Cₛ**aₛtₛtₛgₛgₛtₛaₛt**Tₛ^{Me}CₛA**-3' | 14 | Motif #5 |
| SEQ ID NO: 22 | 5'-**AₛGₛ^{Me}Cₛ**aₛtₛtₛgₛgₛtₛaₛtₛ**T^{Me}CₛA**-3' | 14 | Motif #5 |
| SEQ ID NO: 23 | 5'-**AₛGₛ^{Me}Cₛ**aₛtₛtₛgₛgₛtₛaₛtₛ**T^{Me}ₛCA**-3' | 14 | Motif #5 |
| SEQ ID NO: 24 | 5'-**AGₛ^{Me}Cₛ**aₛtₛtₛgₛgₛtₛaₛtₛ**Tₛ^{Me}CA**-3' | 14 | Motif #5 |
| SEQ ID NO: 25 | 5'-**AG^{Me}Cₛ**aₛtₛtₛgₛgₛtₛaₛtₛ**T^{Me}CA**-3' | 14 | Motif #5 |
| SEQ ID NO: 26 | 5'-**Gₛ^{Me}Cₛ**aₛtₛtₛgₛgₛtₛaₛtₛ**Tₛ^{Me}CₛA**-3' | 13 | Motif #9 |
| SEQ ID NO: 27 | 5'-**G^{Me}Cₛ**a**ₛ**tₛtₛgₛgₛtₛaₛtₛ**Tₛ^{Me}CₛA**-3' | 13 | Motif #9 |
| SEQ ID NO: 28 | 5'-**Gₛ^{Me}C**aₛtₛtₛgₛgₛtₛaₛtₛ**Tₛ^{Me}CₛA**-3' | 13 | Motif #9 |
| SEQ ID NO: 29 | 5'-**Gₛ^{Me}C**ₛaₛtₛtₛgₛgₛtₛaₛt**Tₛ^{Me}CₛA**-3' | 13 | Motif #9 |
| SEQ ID NO: 30 | 5'-**Gₛ^{Me}Cₛ**aₛtₛtₛgₛtₛtₛaₛtₛ**T^{Me}CₛA**-3' | 13 | Motif #9 |
| SEQ ID NO: 31 | 5'-**Gₛ^{Me}Cₛ**aₛtₛtₛgₛgₛtₛaₛtₛ**T^{Me}ₛCA**-3' | 13 | Motif #9 |
| SEQ ID NO: 32 | 5'-**G^{Me}Cₛ**aₛtₛtₛgₛgₛtₛaₛtₛ**Tₛ^{Me}CA**-3' | 13 | Motif #9 |
| SEQ ID NO: 33 | 5'-**G^{Me}Cₛ**aₛtₛtₛgₛgₛtₛaₛtₛ**T^{Me}CA**-3' | 13 | Motif #9 |
| SEQ ID NO: 34 | 5'-**Gₛ^{Me}Cₛ**aₛtₛtₛgₛgₛtₛaₛtₛ**Tₛ^{Me}C**-3' | 12 | Motif #13 |
| SEQ ID NO: 35 | 5'-**G^{Me}Cₛ**aₛtₛtₛgₛgₛtₛaₛtₛ**Tₛ^{Me}C**-3' | 12 | Motif #13 |
| SEQ ID NO: 36 | 5'-**Gₛ^{Me}C**aₛtₛtₛgₛgₛtₛaₛtₛ**Tₛ^{Me}C**-3' | 12 | Motif #13 |
| SEQ ID NO: 37 | 5'-**Gₛ^{Me}Cₛ**aₛtₛtₛgₛgₛtₛaₛt**Tₛ^{Me}C**-3' | 12 | Motif #13 |
| SEQ ID NO: 38 | 5'-**Gₛ^{Me}Cₛ**aₛtₛtₛgₛgₛtₛaₛtₛ**T^{Me}C**-3' | 12 | Motif #13 |
| SEQ ID NO: 39 | 5'-**G^{Me}Cₛ**aₛtₛtₛgₛgₛtₛaₛtₛ**T^{Me}C**-3' | 12 | Motif #13 |
| SEQ ID NO: 40 | 5'-**G^{Me}C**aₛtₛtₛgₛgₛtₛaₛt**T^{Me}C**-3' | 12 | Motif #13 |

### Example 3: Cholesterol levels in plasma

Total cholesterol level is measured in plasma using a colometric assay Cholesterol CP from ABX Pentra. The cholesterol is measured following enzymatic hydrolysis and oxidation. 21.5 µL water was added to 1.5 µL plasma.

250 µL reagent is added and within 5 min the cholesterol content is measured at a wavelength of 540 nM. Measurments on each animal was made in duplicates. The sensitivity and linearity was tested with 2 fold diluted control compound (ABX Pentra N control). The relative Cholesterol level was determined by subtraction of the background and presented relative to the cholesterol levels in plasma of saline treated mice.

### Example 4: Measurments of mRNA levels

Antisense modulation of Apo-8100 expression can be assayed in a variety of ways known in the art. For example, Apo-B100 mRNA levels can be quantitated by, e.g., Northern blot analysis, competitive polymerase chain reaction (PCR), or real-time PCR. Real-time quantitative PCR is presently preferred. RNA analysis can be performed on total cellular RNA or mRNA. Methods of RNA isolation and RNA analysis such as Northern blot analysis is routine in the art and is taught in, for example, Current Protocols in Molecular Biology, John Wiley and Sons.

Real-time quantitative (PCR) can be conveniently accomplished using the commercially iQ Multi-Color Real Time PCR Detection System available from BioRAD. Real-time Quantitative PCR is a technique well known in the art and is taught in for example Heid et al. Real time quantitative PCR, Genome Research (1996), 6: 986-994.

### Example 5: Screening of oligonucleotides targeting ApoB-100 mRNA (dosing 3*5 mg/kg)

In this study 5 mg/kg/dose were dosed on 3 consecutive days (one dose/day i.v.) and animals were sacrificed 24 hours after last dosing. At sacrifice, liver and retro orbital sinus blood was sampled. Serum was prepared from blood for analysis of cholesterol. RNA was isolated from the liver and the expression of ApoB-100 mRNA was measured.

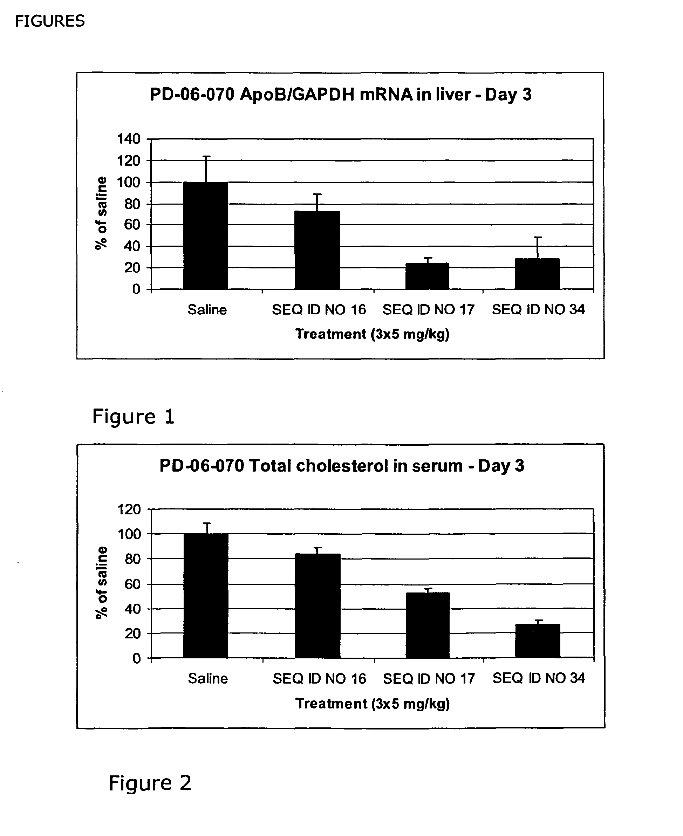

The effect of dosing three doses at 5 mg/kg/dose of oligoes of different length on ApoB-100 mRNA expression is shown in Figure 1. SEQ ID NO 16 down regulated ApoB-100 mRNA with about 25-30%, whereas the 14-mer SEQ ID NO 17 and 12-mer SEQ ID NO 34 was much more potent and equally potent - down regulated ApoB-100 mRNA with about 75% after dosing 3 times 5 mg/kg.

Total cholesterol was measured in serum at sacrifice, day 3 (Figure 2). Similar to the results from the qPCR the best or the most potent effect was obtained with the 12-mer SEQ ID NO 34 followed by the 14-mer SEQ ID NO 17. The 16-mer (SEQ ID NO 16) reduced total cholesterol with about 18% .

### Example 6: Dose response and duration of action of SEQ ID NO 17 and SEQ ID NO 34 in C57BL/6 Female Mice.

In this study three different concentrations (10, 15 and 25 mg/kg) of SEQ ID NO 17 and SEQ ID NO 34 were examined for duration of action on ApoB-100 mRNA expression and serum cholesterol level. SEQ ID NO 17 and SEQ ID NO 34 were given as a single dose of 10, 15 or 25 mg/kg to C57BL/6 female mice. Mice were sacrificed at different time points (1, 3, 5 and 8 days) after dosing; liver and serum were examined for ApoB-100 mRNA expression, liver oligonucleotide concentration and cholesterol and ALT, respectively.

### Analysis of target mRNA down regulation

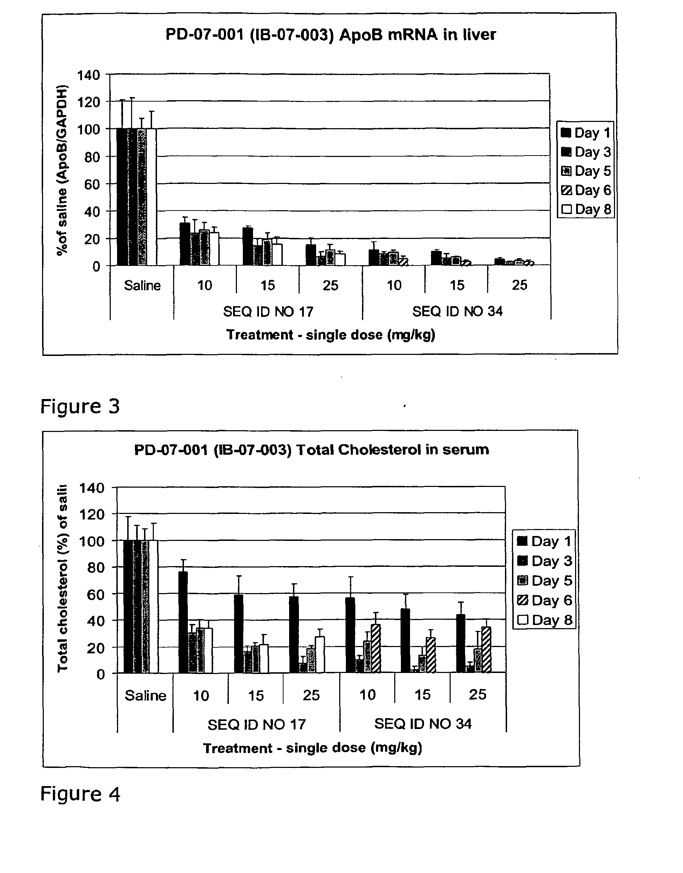

Liver sampled at sacrifice was analysed for ApoB-100 mRNA expression by qPCR. Data was normalized to Gapdh and presented relative to the data obtained by dosing saline. One dose of 10, 15 or 25 mg/kg of SEQ ID NO 17 or SEQ ID NO 34 was very effective to down regulate ApoB-100 mRNA in liver (Figure 3). Twenty-four hours after dosing, down regulation of 90-95 % was obtained with SEQ ID NO 34, whereas dosing of SEQ ID NO 17 resulted in 70-85% lower ApoB mRNA levels than in the saline control group.

### Serum cholesterol

Blood serum used to measure cholesterol was sampled at sacrifice. Twenty-four hours after dosing SEQ ID NO 17 serum total cholesterol was reduced 25-40%, and dosing SEQ ID NO 34 gave 40-55% reduction in total cholesterol. At day 3, the total cholesterol level was further reduced: SEQ ID NO 17 gave 70-90% reduction in a dose dependent manner after doing 10, 15 or 25 mg/kg. SEQ ID NO 34 reduced total cholesterol with 90-95% relative to the saline control group. At day 5 -8 the total cholesterol level increased in all groups except the group dosed SEQ ID NO 17 at 10 mg/kg. (Figure 4.).

### Example 7: Dose response and duration of action of SEQ ID NO 17 and SEQ ID NO 34 in C576L/6 Female Mice

A single dose of SEQ ID NO 17 and SEQ ID NO 34 at different concentrations was administered to C57BL/6J mice to find ED50 values for cholesterol. Duration of action was also included in this study, because we previously have seen that maximum effect of a single dose not always was achieved 24 hours after dosing. In Example 6, we completely down- regulated ApoB-100 mRNA after dosing 10, 15 or 25 mg/kg SEQ ID NO 34 and 25 mg/kg SEQ ID NO 17. In this study we therefore have chosen lower concentrations (1, 2.5 and 5 mg/kg).

### Analysis of target mRNA down regulation

Liver sampled at sacrifice was analysed for ApoB-100 mRNA expression by qPCR. Data was normalized to Gapdh and presented relative to the data obtained by dosing saline. One dose of 10, 15 or 25 mg/kg of SEQ ID NO 17 or SEQ ID NO 34 was very effective to down regulate ApoB-100 mRNA in liver (

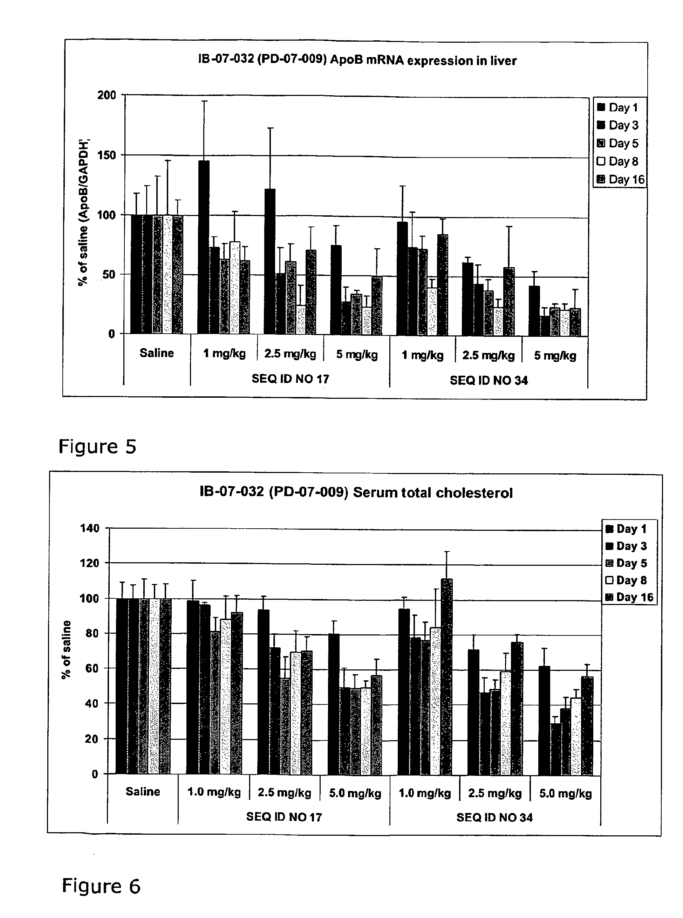

Figure 5). A single dose of SEQ ID NO 17 of 1, 2.5 or 5 mg/kg resulted in a dose dependent down regulation of ApoB-100 mRNA with a duration of 5 days. Similar results were obtained with SEQ ID NO 34. At day 8 both oligonucleotides resulted in ApoB-100 mRNA expression that was similar after dosing 2.5 SEQ ID NO 34 and 5 mg/kg SEQ ID NO 17, reduction of 75%. At day 16 the mRNA level had increased again in all groups, except after dosing 5 mg/kg SEQ ID NO 34 with ApoB-100 mRNA down regulation of 75% similar to that at days 5 and 8.

### Serum cholesterol

Blood serum was sampled at sacrifice and used to measure cholesterol. The serum total cholesterol level reflected the mRNA expression of ApoB-100; dose dependent reduction with best effect at 5 days after dosing SEQ ID NO 17 at 1 and 2.5 mg/kg and similar effect at days 3, 5 and 8 after dosing 5 mg/kg (50% reduction). Dose dependent effect was also obtained after dosing SEQ ID NO 34 with best effect at day 3 after dosing 5 mg/kg (70% reduction) with following increase in cholesterol level (60% reduction at day 8 and 45% at day 16). However, the cholesterol levels in the groups dosed SEQ ID NO 34 did not follow the mRNA reductions in the groups dosed 2.5 and 5 mg/kg, e.g. dosing 5 mg/kg gave about 75% down regulation of ApoB-100 mRNA days 5-16 whereas the cholesterol level after dosing 2.5 mg/kg and 5 mg/kg increased from day 3 to day 16 from a 70% reduction to 45% reduction.

### Example 8: Screening of oligonucleotides targeting ApoB-100 mRNA (dosing 3 * 1 or 5 mg/kg i. v. three consecutive days)

The effect on ApoB-100 mRNA was examined at different days after dosing 1.0 or 5.0 mg/kg (one dose day 0) of the three LNA antisense oligonucleotides 12-mer SEQ ID NO 34, SEQ ID NO 26 13-mer and 14-mer SEQ ID NO 17 all targeting ApoB mRNA.

### Analysis of target mRNA down regulation

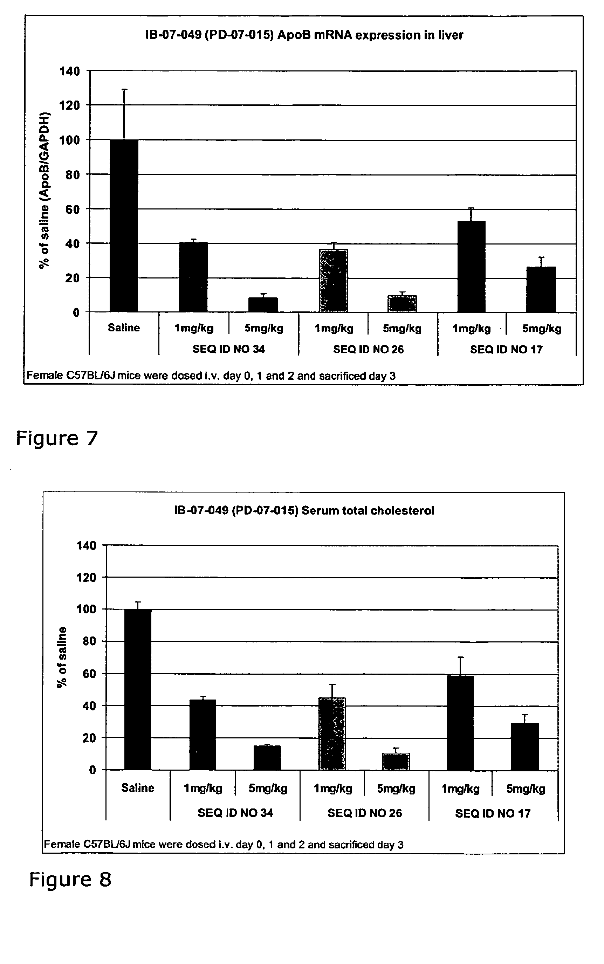

Liver sampled at sacrifice was analysed for ApoB-100 mRNA expression by qPCR. Data was normalized to Gapdh and presented relative to the data obtained by dosing saline. Dosing 3* 1 or 5 mg/kg of SEQ ID NO 34, SEQ ID NO 17 or SEQ ID NO 26 was very effective to down regulate ApoB-100 mRNA in liver (Figure 7). Dosing 1 mg/kg SEQ ID NO 34 or SEQ ID NO 26 down regulated ApoB-100 mRNA with 60% and 5 mg/kg resulted in 90% down regulation similar for both compounds. SEQ ID NO 17 dosed 3* 1 mg/kg/dose or 5 mg/kg/dose down regulated target mRNA with 50% and 70% respectively.

### Serum cholesterol

At sacrifice blood for serum was sampled and used to measure cholesterol. Similar to the results for the mRNA expression, the SEQ ID NO 34 and SEQ ID NO 26 gave similar results: 60% reduction after dosing 3*1 mg/kg and about 85-90% after 3*5 mg/kg/dose. The SEQ ID NO 17 was a little less potent and reduced serum cholesterol with 40% and 70% after dosing 3* 1 or 5 mg/kg/dose, respectively.

### Example 9: Different length (16-mer - 10mer) and LNA design of oligonucleotides targeting ApoB-100 mRNA (dosing 3 * 5 mg/kg i. v. three consecutive days)

In this study 5 mg/kg/dose were dosed on 3 consecutive days (one dose/day i.v.) and animals were sacrificed 24 hours after last dosing. At sacrifice, liver was sampled. RNA was isolated from the liver and the expression of ApoB-100 mRNA was measured using qPCR.

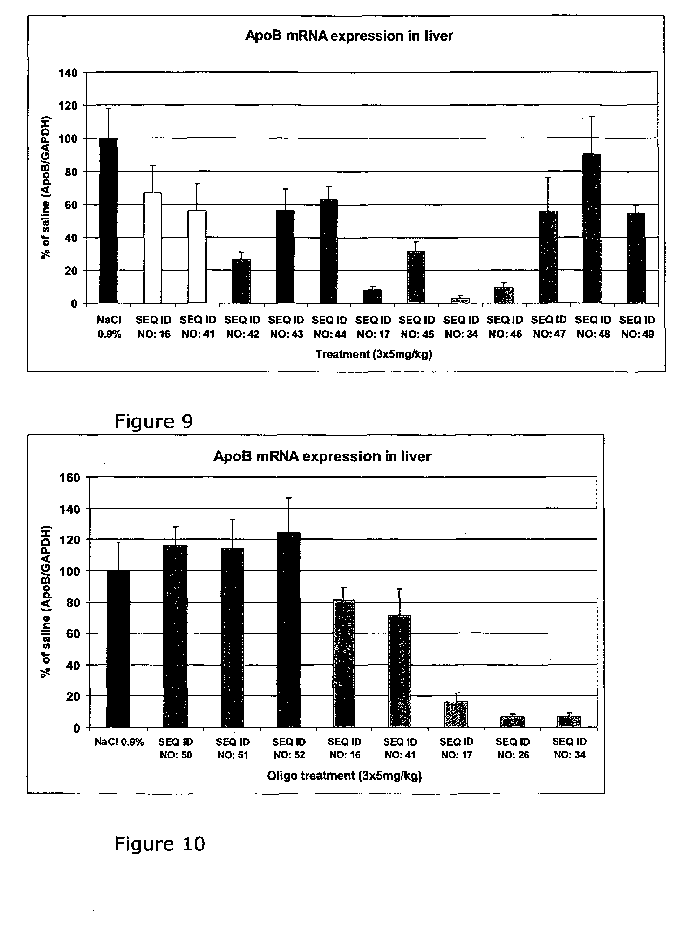

The effect of dosing three doses at 5 mg/kg/dose of oligos of different length on ApoB-100 mRNA expression is shown in (Figure 9). SEQ ID NO 16 and SEQ ID NO 41 down regulated ApoB-100 mRNA with about 35% - 40 %. Between the 14 mers SEQ ID NO 43 and SEQ ID NO 44 down regulated about 35% - 40 %, whereas the 14-mer SEQ ID NO 42 down regulated 70% and SEQ ID NO 17 down regulated 90%. Among the 12-mers SEQ ID NO 34 was the most potent of all the oligos in this study >90 % down regulated whereas SEQ ID NO 45 and SEQ ID NO 46 - down regulated ApoB-100 mRNA with about 70% and 90% respectively. 10 mers showed a maximum of 35% - 40% down regulation.

### Example 10: Different length (16-mer - 12mer) of oligonucleotides targeting ApoB-100 mRNA or Hifl -alpha mRNA (dosing 3 * 5 mg/kg i. v. three consecutive days)

In this study 5 mg/kg/dose were dosed on 3 consecutive days (one dose/day i.v.) and animals were sacrificed 24 hours after last dosing. At sacrifice, liver and retro orbital sinus blood was sampled. Serum was prepared from blood for analysis of cholesterol. RNA was isolated from the liver and the expression of ApoB-100 mRNA and Hif1-alpha was measured using qPCR.

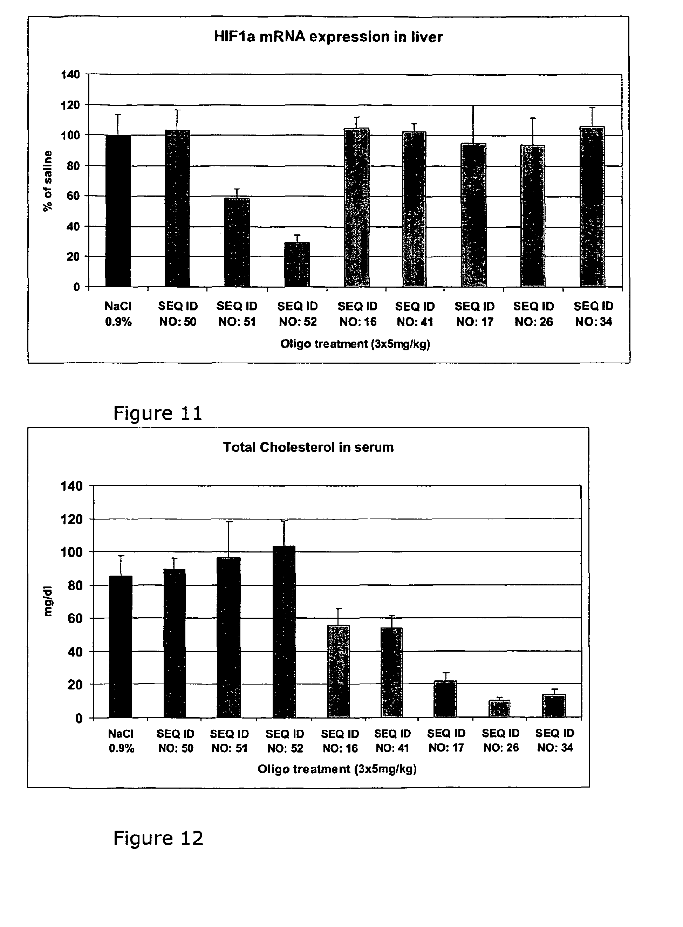

SEQ ID NOS 16, 41, 17 , 26 , 34 all against ApoB mRNA down regulated ApoB 30%, 30%, 80%, 90%, 90% respectively but had no effect on Hif1-alpha mRNA (Figure 10 and Figure 11). SEQ ID NO's 50, 51 and 52 against Hif1-alpha down-regulated Hif1-alpha mRNA by 0%, 40% and 70% respectively but had no effect on ApoB mRNA (Figure 10 and Figure 11).

SEQ ID NOS 16, 41, 17 , 26 , 34 all against ApoB mRNA down regulated serum cholesterol by 40%, 40%, 80%, 90%, 85% respectively whereas SEQ ID NO's 50, 51, 52 showed no effect on serum cholesterol (Figure 12).

### Example 11: Different length (16-mer - 12mer) of oligonucleotides targeting Hif 1-alpha mRNA (dosing 3 * 5 mg/kg i.v. three consecutive days) in liver and kidney

In this study 5 mg/kg/dose were dosed to NMRI mice on 3 consecutive days (one dose/day i.v.) and animals were sacrificed 24 hours after last dosing. At sacrifice, liver and kidney tissue were sampled. RNA was isolated from the tissues and the expression of Hif1-alpha mRNA was measured using qPCR.

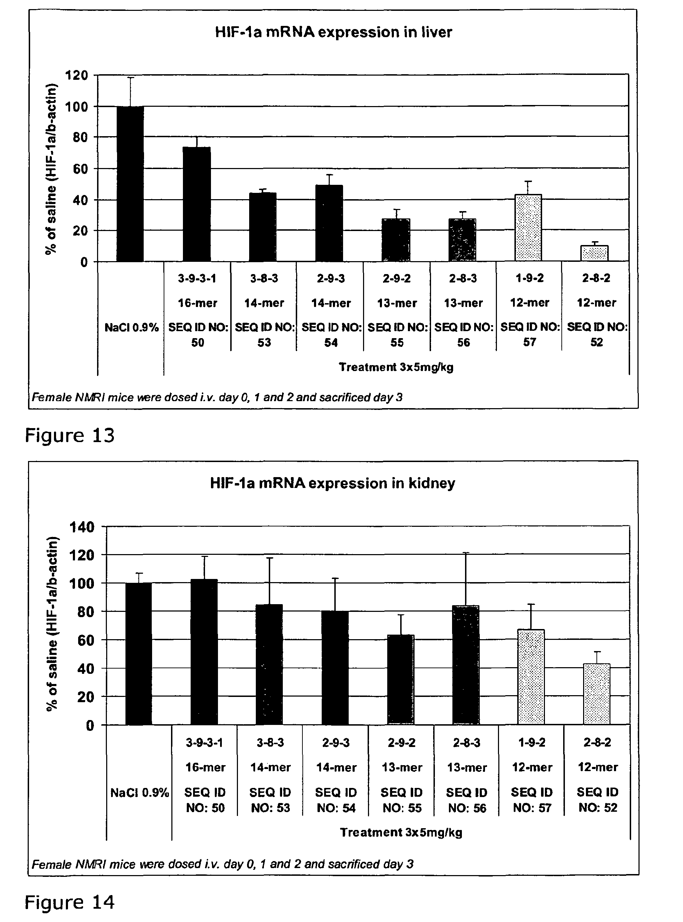

SEQ ID NOS 50, 53, 54, 55,56,57,52 against Hif1- alpha down-regulated Hif1-alpha mRNA by 25%, 55%, 50%, 70%, 70% 58% and 90% respectively in the liver and down-regulated Hif1-alpha mRNA in kidney by 0%, 15%, 20%, 40%, 20%, 30% and 60% respectively. (Figure 13 and 14)

### Example 12: ApoB mRNA knockdown in liver and kidney. Shifting the ApoB mRNA knockdown from liver to kidney.

In this study Female C57BL/6 mice were dosed with either 0.2 mg/kg or 1 mg/kg on 3 consecutive days (one dose/day i.v.) and animals were sacrificed 24 hours after last dosing. At sacrifice, liver and kidney tissue were sampled. RNA was isolated from the tissues and the expression of ApoB mRNA was measured using qPCR.

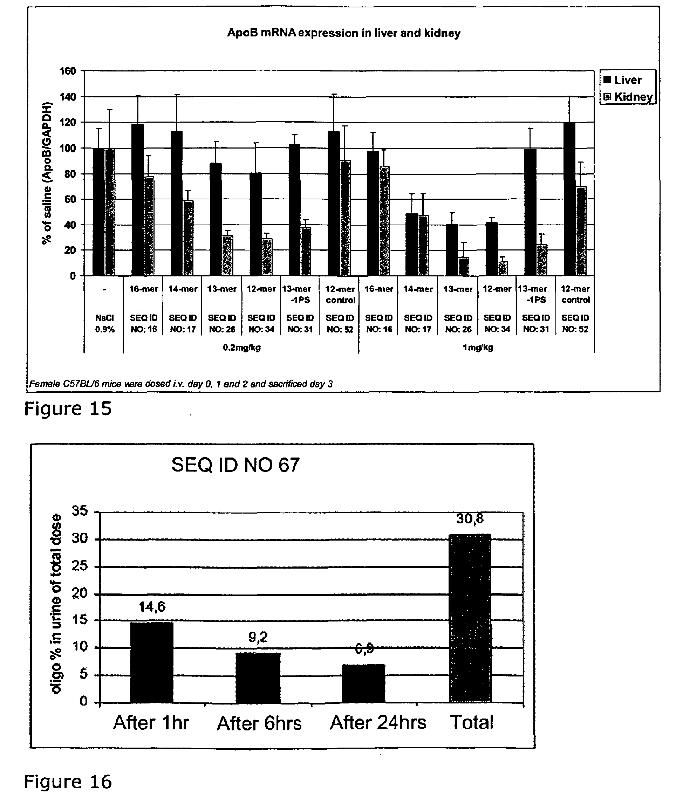

SEQ ID NOS 16, 17, 26, 34, 31, against ApoB mRNA and SEQ ID NO 52 against Hif1-alpha were monitored for their ability to downregulate ApoB mRNA in liver and kidney. (Figure 15). SEQ ID NOS 16, 17, 26, 34, 31, 52 dowregulate ApoB mRNA in liver/kidney 0%/20%, 0%/40%, 10%/70%, 20%/70%, 0%/65%, 0%/0% respectively at 0.2 mg/kg and 0%/5%, 50%/50%, 60%/85%, 60%/90%, 0%/80%, 0%/30% repectively at 1.0 mg/kg. In SEQ ID NO 31 one phosphorthioate has been replaced by a phosphodiester at linkaged 12 from the 5' end. This modification directs the dowregulation of ApoB mRNA with SEQ ID NO 31 towards the kidney (Figure 15).

### Example 13: Comparison of biodistribution of fully phosphorothioate gapmer with equivalent oligomer where two phosphorothioate linkages have been replaced with phosphodiester.

The following oligomers were synthesised:

| **Test substance** | **Target** | **Sequence** |
|---|---|---|
| SEQ ID NO 67 | Hif-1α | TGGCAAGCATCCTGTA (Motif sequence) |
| SEQ ID NO 68 | Hif-1α | 5'-**Tₛ^{o}Gₛ^{o}Gₛ^{o}**cₛaₛaₛgₛcₛaₛtₛcₛcₛ**Tₛ^{o}Gₛ^{o}Tₛ^{o}**a-3' |
| SEQ ID NO 69 | Hif-1α | 5'-**Tₛ^{o}G^{o}Gₛ^{o}**cₛaₛaₛgₛcₛaₛtₛcₛcₛ**T^{o}Gₛ^{o}Tₛ^{o}**a-3' |

The oligomers were injected into mice at a dosage of 50mg/kg. Urine was sampled after I hour, 6 hours and 24 hours. Animals were killed after 24 hours, and the levels of each oligomer present in the liver and kidney was assessed.

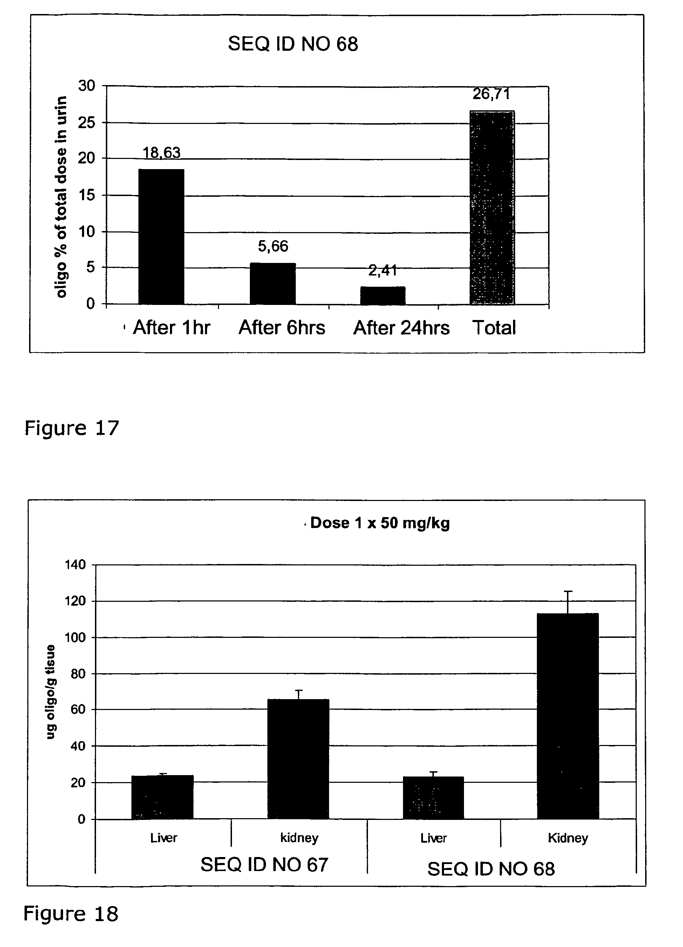

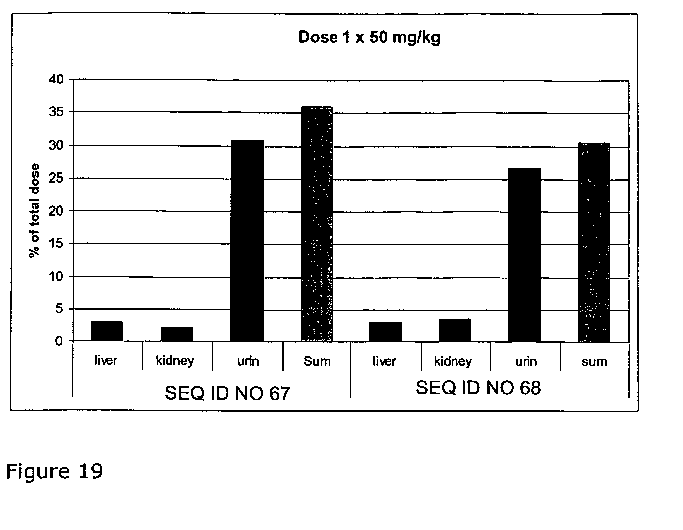

The results are shown in figures 16, 17, 18 and 19.

SEQ ID NO 68 was found to be secreted at a slightly higher rate from the urine over the 24hour period, although the initial rate of excretion appears to be higher with SEQ ID NO 69.

The amount of SEQ ID NO 69 with 2 PO's distributed to the kidney is almost twice as much as SEQ ID NO 68.

SEQ ID NO 69 shows a wider biodistribution to other tissues - 69% of SEQ ID NO 69 distributes to other tissues compared to 64% of SEQ ID NO 68.

## Claims

1. An oligomer consisting of a contiguous nucleobase sequence of a total of between 10 - 20 nucleobases, wherein the contiguous nucleobase sequence is of formula (5' - 3'), A-B-C, or optionally A-B-C-D or D-A-B-C, wherein;
- region A (5' region) consists or comprises of 1-6 LNA units;
- region B (central domain), immediately 3' (i.e. contiguous) to A, consists of between 6 - 12 contiguous nucleobase units which are capable of recruiting RNAseH when formed in a duplex with a complementary RNA molecule (such as a mRNA target), such as DNA units, and;
- region C(3' region) immediately 3' to B, consists or comprises of 1-6 LNA units; and
- region D, when present, consists of one or two DNA units
and wherein said oligomer comprises of a total of 1, or 2 phosphodiester linkages; wherein the phosphodiester bond or bonds are inserted between or adjacent to nucleotide analogue(s) of region A and/or C, and wherein the remaining internucleoside linkages are all phosphorothioate.

2. The oligomer according to claim 1 wherein the oligomer consisting of a contiguous nucleobase sequence of a total of 10, 11, 12, 13, 14, 15 or 16 nucleobase units.

3. The oligomer according to claim 3, wherein;
- region A consists of 1, 2 or 3 LNA units;
- region B consists of 7, 8 or 9 contiguous nucleobase units which are capable of recruiting RNAseH when formed in a duplex with a complementary RNA molecule (such as a mRNA target), preferably DNA units; and
- region C consists of 1, 2 or 3 LNA units;

4. The oligomer according to any one of claims 1 -3, wherein said oligomer consists of a total 10, 11, 12, 13 or 14 nucleobases.

5. The oligomer according to any one of claims 1 - 4, wherein region A comprises at least one phosphodiester linkage between two LNA units, and/or a phosphodiester linkage between the 3' LNA unit of region A and the 5' nucleobase unit of region B.

6. The oligomer according to any one of claims 1 - 5, wherein region C comprises at least one phosphodiester linkage between two LNA units, and/or a phosphodiester linkage between the 5' LNA unit of region C and the 3' nucleobase unit of region B.

7. The oligomer according to any one of claims 3 - 6, wherein the number of nucleobases in regions A, B, and C (A-B-C) are selected from the group consisting of: 1-8-2, 2-8-1, 2-8-2, 3-8-3, 2-8-3, 3-8-2.

8. The oligomer according to any one of claims 3 - 6, wherein the number of nucleobases in regions A, B, and C (A-B-C) are 3-9-3, 3-10-3, 4-8-3, or 3-8-4.

9. A conjugate comprising the oligomer according to any one of the claims 1 - 8 and at least one non-nucleotide or non-polynucleotide moiety covalently attached to said compound.

10. A pharmaceutical composition comprising an oligomer as defined in any of claims 1-8 or a conjugate as defined in claim 9, and a pharmaceutically acceptable diluent, carrier, salt or adjuvant.

11. The the oligomer according to any one of claims 1 - 8, or the conjugate according to claim 9, for use in medicine.

12. An in vitro method for the reduction in the cellular concentration of a mRNA in a mammalian cell, said method comprising the administration of an oligomer according to any one of claims 1 - 8, or the conjugate according to claim 9, to the mammalian cell, wherein said mammalian cell comprises an mRNA species which comprises a nucleobase sequence which is complementary to said oligomer.

## Patentansprüche

1. Oligomer, bestehend aus einer zusammenhängenden Nukleobasensequenz mit insgesamt zwischen 10 und 20 Nukleobasen, wobei die zusammenhängende Nukleobasensequenz die Formel (5' - 3'), A-B-C oder fakultativ A-B-C-D oder D-A-B-C aufweist, wobei;
- Region A (5'-Region) aus 1-6 LNA-Einheiten besteht oder diese umfasst;
- Region B (zentrale Domäne), unmittelbar 3' zu (d. h. zusammenhängend mit) A, aus 6 - 12 zusammenhängenden Nukleobaseneinheiten besteht, die RNAseH rekrutieren können, wenn sie mit einem komplementären RNA-Molekül (wie einem mRNA-Ziel) einen Duplex bilden, wie DNA-Einheiten, und;
- Region C (3'-Region), unmittelbar 3' zu B, aus 1-6 LNA-Einheiten besteht oder diese umfasst; und
- Region D, sofern vorhanden, aus einer oder zwei DNA-Einheiten besteht;
und wobei das Oligomer insgesamt 1 oder 2 Phosphodiester-Bindungen umfasst; wobei die Phosphodiester-Bindung bzw. -Bindungen zwischen oder neben Nukleotidanalogon(s) von Region A und/oder C eingesetzt sind und wobei die übrigen Internukleosidbindungen alle Phosphorthioat sind.

2. Oligomer nach Anspruch 1, wobei das Oligomer aus einer zusammenhängenden Nukleobasensequenz mit insgesamt 10, 11, 12, 13, 14, 15 oder 16 Nukleobaseneinheiten besteht.

3. Oligomer nach Anspruch 3, wobei;
- Region A aus 1, 2 oder 3 LNA-Einheiten besteht;
- Region B aus 7, 8 oder 9 zusammenhängenden Nukleobaseneinheiten besteht, die RNAseH rekrutieren können, wenn sie mit einem komplementären RNA-Molekül (wie einem mRNA-Ziel) einen Duplex bilden, vorzugsweise DNA-Einheiten; und
- Region C aus 1, 2 oder 3 LNA-Einheiten besteht;

4. Oligomer nach einem der Ansprüche 1 -3, wobei das Oligomer aus insgesamt 10, 11, 12, 13 oder 14 Nukleobasen besteht.

5. Oligomer nach einem der Ansprüche 1 - 4, wobei Region A mindestens eine Phosphodiester-Bindung zwischen zwei LNA-Einheiten und/oder eine Phosphodiester-Bindung zwischen der 3'-LNA-Einheit von Region A und der 5'-Nukleobaseneinheit von Region B umfasst.

6. Oligomer nach einem der Ansprüche 1 - 5, wobei Region C mindestens eine Phosphodiester-Bindung zwischen zwei LNA-Einheiten und/oder eine Phosphodiester-Bindung zwischen der 5'-LNA-Einheit von Region C und der 3'-Nukleobaseneinheit von Region B umfasst.

7. Oligomer nach einem der Ansprüche 3 - 6, wobei die Anzahl der Nukleobasen in Region A, B und C (A-B-C) ausgewählt ist aus der Gruppe, bestehend aus: 1-8-2, 2-8-1, 2-8-2, 3-8-3, 2-8-3, 3-8-2.

8. Oligomer nach einem der Ansprüche 3 - 6, wobei die Anzahl der Nukleobasen in Region A, B und C (A-B-C) 3-9-3, 3-10-3, 4-8-3 oder 3-8-4 beträgt.

9. Konjugat, umfassend das Oligomer nach einem der Ansprüche 1 - 8 und mindestens eine Nichtnukleotid- oder Nichtpolynukleotid-Gruppierung, die kovalent an die Verbindung gebunden ist.

10. Pharmazeutische Zusammensetzung, umfassend ein Oligomer nach einem der Ansprüche 1-8 oder ein Konjugat nach Anspruch 9 und ein pharmazeutisch unbedenkliches Verdünnungsmittel, Trägermittel, Salz oder Adjuvans.

11. Oligomer nach einem der Ansprüche 1 - 8 oder Konjugat nach Anspruch 9 zur Verwendung in der Medizin.

12. In-vitro-Verfahren zur Senkung der Zellkonzentration einer mRNA in einer Säugetierzelle, wobei das Verfahren die Verabreichung eines Oligomers nach einem der Ansprüche 1 - 8 oder des Konjugats nach Anspruch 9 an die Säugetierzelle umfasst, wobei die Säugetierzelle eine mRNA-Spezies umfasst, die eine dem Oligomer komplementäre Nukleobasensequenz umfasst.

## Revendications

1. Oligomère constitué d'une séquence de nucléobases contiguës d'un total de 10 à 20 nucléobases, dans lequel la séquence de nucléobases contiguës est de formule (5' - 3'), A-B-C ou éventuellement A-B-C-D ou D-A-B-C, dans laquelle :
- la région A (région 5') est constituée de ou comprend 1 à 6 unités LNA ;
- la région B (domaine central), immédiatement 3' (c.-à-d. contiguë) à A, est constituée de 6 à 12 unités nucléobase contiguës qui sont capables de recruter la RNAseH lorsqu'elles forment un duplex avec une molécule d'ARN complémentaire (telle qu'un ARMm cible), telles que des unités d'ADN, et ;
- la région C (région 3'), immédiatement 3' à B, est constituée de ou comprend 1 à 6 unités LNA ; et
- la région D, lorsqu'elle est présente, est constituée d'une ou de deux unités d'ADN ;
et dans lequel ledit oligomère comprend un total de 1 ou 2 liaisons phosphodiester ; dans lequel la ou les liaisons phosphodiester sont insérées entre ou adjacentes à un(des) analogue(s) nucléotidique(s) de la région A et/ou C, et dans lequel les liaisons internucléosidiques restantes sont toutes de type phosphorothioate.

2. L'oligomère selon la revendication 1, dans lequel l'oligomère est constitué d'une séquence de nucléobases contiguës d'un total de 10, 11, 12, 13, 14, 15 ou 16 unités nucléobase.

3. L'oligomère selon la revendication 3, dans lequel :
- la région A est constituée de 1, 2 ou 3 unités LNA ;
- la région B est constituée de 7, 8 ou 9 unités nucléobase contiguës qui sont capables de recruter la RNAseH lorsqu'elles forment un duplex avec une molécule d'ARN complémentaire (telle qu'un ARMm cible), de préférence des unités d'ADN ; et
- la région C est constituée de 1, 2 ou 3 unités LNA.

4. L'oligomère selon l'une quelconque des revendications 1 à 3, dans lequel ledit oligomère est constitué d'un total de 10, 11, 12, 13 ou 14 nucléobases.

5. L'oligomère selon l'une quelconque des revendications 1 à 4, dans lequel la région A comprend au moins une liaison phosphodiester entre deux unités LNA, et/ou une liaison phosphodiester entre l'unité LNA 3' de la région A et l'unité nucléobase 5' de la région B.

6. L'oligomère selon l'une quelconque des revendications 1 à 5, dans lequel la région C comprend au moins une liaison phosphodiester entre deux unités LNA, et/ou une liaison phosphodiester entre l'unité LNA 5' de la région C et l'unité nucléobase 3' de la région B.

7. L'oligomère selon l'une quelconque des revendications 3 à 6, dans lequel le nombre de nucléobases dans les régions A, B et C (A-B-C) est choisi dans le groupe consistant en : 1-8-2, 2-8-1, 2-8-2, 3-8-3, 2-8-3, 3-8-2.

8. L'oligomère selon l'une quelconque des revendications 3 à 6, dans lequel le nombre de nucléobases dans les régions A, B et C (A-B-C) est 3-9-3, 3-10-3, 4-8-3 ou 3-8-4.

9. Conjugué comprenant l'oligomère selon l'une quelconque des revendications 1 à 8 et au moins une fraction non nucléotidique ou non polynucléotidique fixée par liaison covalente au dit composé.

10. Composition pharmaceutique comprenant un oligomère tel que défini dans l'une quelconque des revendications 1 à 8 ou un conjugué tel que défini dans la revendication 9, et un diluant, un véhicule, un sel ou un adjuvant pharmaceutiquement acceptable.

11. L'oligomère selon l'une quelconque des revendications 1 à 8, ou le conjugué selon la revendication 9, destiné à un usage médical.

12. Procédé in vitro destiné à réduire la concentration cellulaire d'un ARNm dans une cellule de mammifère, ledit procédé comprenant l'administration à la cellule de mammifère d'un oligomère selon l'une quelconque des revendications 1 à 8, ou du conjugué selon la revendication 9, dans lequel ladite cellule de mammifère comprend une espèce d'ARNm qui comprend une séquence de nucléobases qui est complémentaire dudit oligomère.


### SEQUENCE LISTING

<110> Santaris A/S
<120> SHORT RNA ANTAGONIST COMPOUNDS FOR THE MODULATION OF TARGET mRNA
<130> 17078PCT00
<150> US 60/896,419
   <151> 2007-03-22
<150> US 60/977,409
   <151> 2007-10-04
<150> US 60/969,016
   <151> 2007-08-30
<150> US 60/990,125
   <151> 2007-11-26
<150> US 60/992,050
   <151> 2007-12-03
<150> US 61/012,191
   <151> 2007-12-07
<150> US 61/012,185
   <151> 2007-12-07
<150> US 61/023,244
   <151> 2008-01-24
<150> US 61/023,250
   <151> 2008-01-24
<160> 65
<170> PatentIn version 3.3
<210> 1
   <211> 16
   <212> DNA
   <213> Artificial
<220>
   <223> Antisense motif
<4.00> 1
   cagcattggt attcag 16
<210> 2
   <211> 15
   <212> DNA
   <213> Artificial
<220>
   <223> Antisense motif
<400> 2
   cagcattggt attca 15
<210> 3
   <211> 15
   <212> DNA
   <213> Artificial
<220>
   <223> Antisense motif
<400> 3
   agcattggta ttcag 15
<210> 4
   <211> 14
   <212> DNA
   <213> Artificial
<220>
   <223> Antisense motif
<400> 4
   cagcattggt attc 14
<210> 5
   <211> 14
   <212> DNA
   <213> Artificial
<220>
   <223> Antisense motif
<400> 5
   agcattggta ttca 14
<210> 6
   <211> 14
   <212> DNA
   <213> Artificial
<220>
   <223> Antisense motif
<400> 6
   gcattggtat tcag 14
<210> 7
   <211> 13
   <212> DNA
   <213> Artificial
<220>
   <223> Antisense motif
<400> 7
   cagcattggt att 13
<210> 8
   <211> 13
   <212> DNA
   <213> Artificial
<220>
   <223> Antisense motif
<400> 8
   agcattggta ttc 13
<210> 9
   <211> 13
   <212> DNA
   <213> Artificial
<220>
   <223> Antisense motif
<400> 9
   gcattggtat tca 13
<210> 10
   <211> 13
   <212> DNA
   <213> Artificial
<220>
   <223> Antisense motif
<400> 10
   cattggtatt cag 13
<210> 11
   <211> 12
   <212> DNA
   <213> Artificial
<220>
   <223> Antisense motif
<400> 11
   cagcattggt at 12
<210> 12
   <211> 12
   <212> DNA
   <213> Artificial
<220>
   <223> Antisense motif
<400> 12
   agcattggta tt 12
<210> 13
   <211> 12
   <212> DNA
   <213> Artificial
<220>
   <223> Antisense motif
<400> 13
   gcattggtat tc 12
<210> 14
   <211> 12
   <212> DNA
   <213> Artificial
<220>
   <223> Antisense motif
<400> 14
   cattggtatt ca 12
<210> 15
   <211> 12
   <212> DNA
   <213> Artificial
<220>
   <223> Antisense motif
<400> 15
   attggtattc ag 12
<210> 16
   <211> 15
   <212> DNA
   <213> Artificial
<220>
   <223> LNA oligomeric compound
<220>
   <221> misc_feature
   <222> (1)..(15)
   <223> Phosphorothioate linkages
<220>
   <221> misc_feature
   <222> (1)..(3)
   <223> LNA units
<220>
   <221> misc_feature
   <222> (3) .. (3)
   <223> 5 methyl cytosine
<220>
   <221> misc_feature
   <222> (13)..(15)
   <223> LNA units
<220>
   <221> misc_feature
   <222> (13) .. (13)
   <223> 5 methyl cytosine
<400> 16
   agcattggta ttcag 15
<210> 17
   <211> 14
   <212> DNA
   <213> Artificial
<220>
   <223> LNA oligomeric compound
<220>
   <221> misc_feature
   <222> (1) .. (14)
   <223> Phosphorothioate linkages
<220>
   <221> misc_feature
   <222> (1) .. (3)
   <223> LNA units
<220>
   <221> misc_feature
   <222> (3) .. (3)
   <223> 5 methyl cytosine
<220>
   <221> misc_feature
   <222> (12) .. (14)
   <223> LNA units
<220>
   <221> misc_feature
   <222> (13) .. (13)
   <223> 5 methyl cytosine
<400> 17
   agcattggta ttca 14
<210> 18
   <211> 14
   <212> DNA
   <213> Artificial
<220>
   <223> LNA oligomeric compound
<220>
   <221> misc feature
   <222> (1) .. (3)
   <223> LNA units
<220>
   <221> misc feature
   <222> (2) .. (19)
   <223> Phosphorothioate linkages
<220>
   <221> misc feature
   <222> (3)..(3)
   <223> 5 methyl cytosine
<220>
   <221> misc feature
   <222> (12)..(14)
   <223> LNA units
<220>
   <221> misc feature
   <222> (13)..(13)
   <223> 5 methyl cytosine
<400> 18
   agcattggta ttca 14
<210> 19
   <211> 14
   <212> DNA
   <213> Artificial
<220>
   <223> LNA oligomeric compound
<220>
   <221> misc_feature
   <222> (1) .. (2)
   <223> Phosphorothioate linkage
<220>
   <221> misc_feature
   <222> (1) .. (3)
   <223> LNA units
<220>
   <221> misc_feature
   <222> (3)..(14)
   <223> Phosphorothioate linkage
<220>
   <221> misc_feature
   <222> (3) .. (3)
   <223> 5 methyl cytosine
<220>
   <221> misc_feature
   <222> (12) .. (14)
   <223> LNA units
<220>
   <221> misc_feature
   <222> (13) .. (13)
   <223> 5 methyl cytosine
<400> 19
   agcattggta ttca 14
<210> 20
   <211> 14
   <212> DNA
   <213> Artificial
<220>
   <223> LNA oligomeric compound
<220>
   <221> misc feature
   <222> (1)..(3)
   <223> Phosphorothioate linkages
<220>
   <221> misc_feature
   <222> (1) .. (3)
   <223> LNA units
<220>
   <221> misc_feature
   <222> (3) .. (3)
   <223> 5 methyl cytosine
<220>
   <221> misc_feature
   <222> (4) .. (14)
   <223> Phosphorothioate linkages
<220>
   <221> misc feature
   <222> (12)..(14)
   <223> LNA units
<400> 20
   agcattggta ttca 14
<210> 21
   <211> 14
   <212> DNA
   <213> Artificial
<220>
   <223> LNA oligomeric compound
<220>
   <221> misc_feature
   <222> (1) .. (14)
   <223> Phosphorothioate linkages
<220>
   <221> misc feature
   <222> (1)..(3)
   <223> LNA units
<220>
   <221> misc_feature
   <222> (3) .. (3)
   <223> 5 methyl cytosine
<220>
   <221> misc_feature
   <222> (12) .. (14)
   <223> LNA units
<220>
   <221> misc feature
   <222> (13) .. (13)
   <223> 5 methyl cytosine
<400> 21
   agcattggta ttca 14
<210> 22
   <211> 14
   <212> DNA
   <213> Artificial
<220>
   <223> LNA oligomeric compound
<220>
   <221> misc_feature
   <222> (1) .. (12)
   <223> Phosphorothioate linkages
<220>
   <221> misc_feature
   <222> (1) .. (3)
   <223> LNA units
<220>
   <221> misc_feature
   <222> (3) .. (3)
   <223> 5 methyl cytosine
<220>
   <221> misc_feature
   <222> (12) .. (14)
   <223> LNA units
<220>
   <221> misc_feature
   <222> (13) .. (14)
   <223> Phosphorothioate linkages
<220>
   <221> misc_feature
   <222> (13) .. (13)
   <223> 5 methyl cytosine
<400> 22
   agcattggta ttca 14
<210> 23
   <211> 14
   <212> DNA
   <213> Artificial
<220>
   <223> LNA oligomeric compound
<220>
   <221> misc_feature
   <222> (1) .. (12)
   <223> Phosphorothioate linkages
<220>
   <221> misc_feature
   <222> (1) .. (3)
   <223> LNA units
<220>
   <221> misc_feature
   <222> (3) .. (3)
   <223> 5 methyl cytosine
<220>
   <221> misc_feature
   <222> (12) .. (14)
   <223> LNA units
<220>
   <221> misc_feature
   <222> (13) .. (14)
   <223> Phosphorothioate linkages
<220>
   <221> misc_feature
   <222> (13) .. (13)
   <223> 5 methyl cytosine
<400> 23
   agcattggta ttca 14
<210> 24
   <211> 14
   <212> DNA
   <213> Artificial
<220>
   <223> LNA oligomeric compound
<220>
   <221> misc feature
   <222> (1)..(3)
   <223> LNA units
<220>
   <221> misc feature
   <222> (2) .. (13)
   <223> Phosphorothioate linkages
<220>
   <221> misc feature
   <222> (3) .. (3)
   <223> 5 mthyl cytosine
<220>
   <221> misc_feature
   <222> (12) .. (14)
   <223> LNA units
<220>
   <221> misc_feature
   <222> (13) .. (13)
   <223> 5 methyl cytosine
<400> 24
   agcattggta ttca 14
<210> 25
   <211> 14
   <212> DNA
   <21.3> Artificial
<220>
   <223> LNA oligomeric compound
<220>
   <221> misc_feature
   <222> (1) .. (3)
   <223> LNA units
<220>
   <221> misc_feature
   <222> (3) .. (12)
   <223> Phosphorothioate linkages
<220>
   <221> misc_feature
   <222> (3) .. (3)
   <223> 5 methyl cytosine
<220>
   <221> misc_feature
   <222> (12) .. (14)
   <223> LNA units
<220>
   <221> misc_feature
   <222> (13) .. (13)
   <223> 5 methyl cytosine
<400> 25
   agcattggta ttca 14
<210> 26
   <211> 13
   <212> DNA
   <213> Artificial
<220>
   <223> LNA oligomeric compound
<220>
   <221> misc_feature
   <222> (1) .. (13)
   <223> Phosphorothioate linkages
<220>
   <221> misc feature
   <222> (1) .. (2)
   <223> LNA units
<220>
   <221> misc feature
   <222> (2) .. (2)
   <223> 5 methyl cytosine
<220>
   <221> misc_feature
   <222> (11) .. (13)
   <223> LNA units
<220>
   <221> misc_feature
   <222> (12) .. (12)
   <223> 5 methyl cytosine
<400> 26
   gcattggtat tca 13
<210> 27
   <211> 13
   <212> DNA
   <213> Artificial
<220>
   <223> LNA oligomeric compound
<220>
   <221> misc_feature
   <222> (1) .. (2)
   <223> LNA units
<220>
   <221> misc_feature
   <222> (2) .. (13)
   <223> Phosphorothioate linkage
<220>
   <221> misc_feature
   <222> (2) .. (2)
   <223> 5 methyl cytosine
<220>
   <221> misc_feature
   <222> (11) .. (13)
   <223> LNA units
<220>
   <221> misc_feature
   <222> (12) .. (12)
   <223> 5 methyl cytosine
<400> 27
   gcattggtat tca 13
<210> 28
   <211> 13
   <212> DNA
   <213> Artificial
<220>
   <223> LNA oligomeric compound
<220>
   <221> misc_feature
   <222> (1) .. (2)
   <223> Phosphorothioate linkage
<220>
   <221> misc_feature
   <222> (1) .. (2)
   <223> LNA units
<220>
   <221> misc_feature
   <222> (2) .. (2)
   <223> 5 methyl cytosine
<220>
   <221> misc_feature
   <222> (3)..(13)
   <223> Phosphorothioate linkage
<220>
   <221> misc_feature
   <222> (11)..(13)
   <223> LNA units
<220>
   <221> misc_feature
   <222> (12) .. (12)
   <223> 5 methyl cytosine
<400> 28
   gcattggtat tca 13
<210> 29
   <211> 13
   <212> DNA
   <213> Artificial
<220>
   <223> LNA oligomeric compound
<220>
   <221> misc_feature
   <222> (1) .. (13)
   <223> Phosphorothioate linkages
<220>
   <221> misc_feature
   <222> (1) .. (2)
   <223> LNA units
<220>
   <221> misc_feature
   <222> (2) .. (2)
   <223> 5 methyl cytosine
<220>
   <221> misc_feature
   <222> (11) .. (13)
   <223> LNA units
<220>
   <221> misc_feature
   <222> (12) .. (12)
   <223> 5 methyl cytosine
<400> 29
   gcattggtat tca 13
<210> 30
   <211> 13
   <212> DNA
   <213> Artificial
<220>
   <223> LNA oligomeric compound
<220>
   <221> misc feature
   <222> (1)..(11)
   <223> Phosphorothioate linkages
<220>
   <221> misc_feature
   <222> (1) .. (2)
   <223> LNA units
<220>
   <221> misc_feature
   <222> (2) .. (2)
   <223> 5 methyl cytosine
<220>
   <221> misc_feature
   <222> (11)..(13)
   <223> LNA units
<220>
   <221> misc_feature
   <222> (12) .. (13)
   <223> Phosphorothioate linkage
<220>
   <221> misc_feature
   <222> (12) .. (12)
   <223> 5 methyl cytosine
<400> 30
   gcattggtat tca 13
<210> 31
   <211> 13
   <212> DNA
   <213> Artificial
<220>
   <223> LNA oligomeric compound
<220>
   <221> misc_feature
   <222> (1) .. (12)
   <223> Phosphorothioate linkages
<220>
   <221> misc_feature
   <222> (1) .. (2)
   <223> LNA units
<220>
   <221> misc_feature
   <222> (2) .. (2)
   <223> 5 methyl cytosine
<220>
   <221> misc_feature
   <222> (11)..(13)
   <223> LNA units
<220>
   <221> misc_feature
   <222> (12) .. (12)
   <223> 5 methyl cytosine
<400> 31
   gcattggtat tca 13
<210> 32
   <211> 13
   <212> DNA
   <213> Artificial
<220>
   <223> LNA oligomeric compound
<220>
   <221> misc_feature
   <222> (1) .. (2)
   <223> LNA units
<220>
   <221> misc_feature
   <222> (2) .. (12)
   <223> Phosphorothioate linkages
<220>
   <221> misc_feature
   <222> (2) .. (2)
   <223> 5 methyl cytosine
<220>
   <221> misc_feature
   <222> (12)..(13)
   <223> LNA units
<220>
   <221> misc_feature
   <222> (12) .. (12)
   <223> 5 methyl cytosine
<400> 32
   gcattggtat tca 13
<210> 33
   <211> 12
   <212> DNA
   <213> Artificial
<220>
   <223> LNA oligomeric compound
<220>
   <221> misc feature
   <222> (1)..(2)
   <223> LNA units
<220>
   <221> misc feature
   <222> (2) .. (11)
   <223> Phosphorothioate linkages
<220>
   <221> misc feature
   <222> (2)..(2)
   <223> 5 methyl cytosine
<220>
   <221> misc feature
   <222> (11) .. (13)
   <223> LNA units
<220>
   <221> misc_feature
   <222> (12) .. (12)
   <223> 5 methyl cytosine
<400> 33
   gcattggtat tc 12
<210> 34
   <211> 12
   <212> DNA
   <213> Artificial
<220>
   <223> LNA oligomeric compound
<220>
   <221> misc feature
   <222> (1) .. (12)
   <223> Phosphorothioate linkages
<220>
   <221> misc_feature
   <222> (1) .. (2)
   <223> LNA units
<220>
   <221> misc_feature
   <222> (2) .. (2)
   <223> 5 methl cytosine
<220>
   <221> misc_feature
   <222> (11) .. (12)
   <223> LNA units
<220>
   <221> misc_feature
   <222> (12) .. (12)
   <223> 5 methl cytosine
<400> 34
   gcattggtat tc 12
<210> 35
   <211> 12
   <212> DNA
   <213> Artificial
<220>
   <223> LNA oligomeric compound
<220>
   <221> misc_feature
   <222> (1) .. (2)
   <223> LNA units
<220>
   <221> misc_feature
   <222> (2) .. (11)
   <223> Phosphorothioate linkages
<220>
   <221> misc_feature
   <222> (2) .. (2)
   <223> 5 methyl cytosine
<220>
   <221> misc_feature
   <222> (11) .. (12)
   <223> LNA units
<220>
   <221> misc_feature
   <222> (12) .. (12)
   <223> 5 methyl cytosine
<400> 35
   gcattggtat tc 12
<210> 36
   <211> 12
   <212> DNA
   <213> Artificial
<220>
   <223> LNA oligomeric compound
<220>
   <221> misc_feature
   <222> (1) .. (2)
   <223> Phosphorothioate linkages
<220>
   <221> misc feature
   <222> (1) .. (2)
   <223> LNA units
<220>
   <221> misc feature
   <222> (2) .. (2)
   <223> 5 methyl cytosine
<220>
   <221> misc_feature
   <222> (3) .. (12)
   <223> Phosphorothioate linkages
<220>
   <221> misc_feature
   <222> (11) .. (12)
   <223> LNA units
<220>
   <221> misc feature
   <222> (12)..(12)
   <223> 5 methyl cytosine
<400> 36
   gcattggtat tc 12
<210> 37
   <211> 12
   <212> DNA
   <213> Artificial
<220>
   <223> LNA oligomeric compound
<220>
   <221> misc_feature
   <222> (1) .. (12)
   <223> Phosphorothioate linkages
<220>
   <221> misc_feature
   <222> (1)..(2)
   <223> LNA units
<220>
   <221> misc_feature
   <222> (2) .. (2)
   <223> 5 methyl cytosine
<220>
   <221> misc_feature
   <222> (11) .. (12)
   <223> LNA units
<220>
   <221> misc_feature
   <222> (12) .. (12)
   <223> 5 methyl cytosine
<400> 37
   gcattggtat tc 12
<210> 38
   <211> 12
   <212> DNA
   <213> Artificial
<220>
   <223> LNA oligomeric compound
<220>
   <221> misc_feature
   <222> (1) .. (11)
   <223> Phosphorothioate linkages
<220>
   <221> misc_feature
   <222> (1) .. (2)
   <223> LNA units
<220>
   <221> misc_feature
   <222> (2) .. (2)
   <223> 5 methyl cytosine
<220>
   <221> misc_feature
   <222> (11) .. (12)
   <223> LNA units
<220>
   <221> misc_feature
   <222> (12) .. (12)
   <223> 5 methyl cytosine
<400> 38
   gcattggtat tc 12
<210> 39
   <211> 12
   <212> DNA
   <213> Artificial
<220>
   <223> LNA oligomeric compound
<220>
   <221> misc_feature
   <222> (1) .. (2)
   <223> LNA units
<220>
   <221> misc_feature
   <222> (2) .. (11)
   <223> Phosphorothioate linkages
<220>
   <221> misc_feature
   <222> (2) .. (2)
   <223> 5 methyl cytosine
<220>
   <221> misc_feature
   <222> (11) .. (12)
   <223> LNA units
<220>
   <221> misc_feature
   <222> (12) .. (12)
   <223> 5 methyl cytosine
<400> 39
   gcattggtat tc 12
<210> 40
   <211> 12
   <212> DNA
   <213> Artificial
<220>
   <223> LNA oligomeric compound
<220>
   <221> misc_feature
   <222> (1) .. (2)
   <223> LNA units
<220>
   <221> misc_feature
   <222> (2) .. (2)
   <223> 5 methyl cytosine
<220>
   <221> misc_feature
   <222> (3) .. (10)
   <223> Phosphorothioate linkages
<220>
   <221> misc_feature
   <222> (11) .. (12)
   <223> LNA units
<220>
   <221> misc feature
   <222> (12)..(12)
   <223> 5 methyl cytosine
<400> 40
   gcattggtat tc 12
<210> 41
   <211> 14
   <212> DNA
   <213> Artificial
<220>
   <223> Hif 1 alpha oligomer sequence motif
<400> 41
   ggcaagcatc ctgt 14
<210> 42
   <211> 14
   <212> DNA
   <213> Artificial
<220>
   <223> Hif 1 alpha oligomer sequence motif
<400> 42
   ggcaagcatc ctgt 14
<210> 43
   <211> 14
   <212> DNA
   <213> Artificial
<220>
   <223> Hif 1 alpha oligomer sequence motif
<400> 43
   ggcaagcatc ctgt 14
<210> 44
   <211> 13
   <212> DNA
   <213> Artificial
<220>
   <223> Hif 1 alpha oligomer sequence motif
<400> 44
   ggcaagcatc ctg 13
<210> 45
   <211> 13
   <212> DNA
   <213> Artificial
<220>
   <223> Hif 1 alpha oligomer sequence motif
<400> 45
   ggcaagcatg ctg 13
<210> 46
   <211> 13
   <212> DNA
   <213> Artificial
<220>
   <223> Hif 1 alpha oligomer sequence motif
<400> 46
   gcaagcatcc tgt 13
<210> 47
   <211> 13
   <212> DNA
   <213> Artificial
<220>
   <223> Hif 1 alpha oligomer sequence motif
<400> 47
   gcaagcatcc tgt 13
<210> 48
   <211> 12
   <212> DNA
   <213> Artificial
<220>
   <223> Hif 1 alpha oligomer sequence motif
<400> 48
   gcaagcatcc tg 12
<210> 49
   <211> 12
   <212> DNA
   <213> Artificial
<220>
   <223> Hif 1 alpha oligomer sequence motif
<400> 49
   gcaagcatcc tg 12
<210> 50
   <211> 12
   <212> DNA
   <213> Artificial
<220>
   <223> Hif 1 alpha oligomer sequence motif
<400> 50
   gcaagcatcc tg 12
<210> 51
   <211> 14
   <212> DNA
   <213> Artificial
<220>
   <223> Hif 1 alpha oligomer sequence motif
<400> 51
   ggcaagcatc ctgt 14
<210> 52
   <211> 13
   <212> DNA
   <213> Artificial
<220>
   <223> Hif 1 alpha oligomer sequence motif
<400> 52
   ggcaagcatc ctg 13
<210> 53
   <211> 13
   <212> DNA
   <213> Artificial
<220>
   <223> Hif 1 alpha oligomer sequence motif
<400> 53
   gcaagcatcc tgt 13
<210> 54
   <211> 12
   <212> DNA
   <213> Artificial
<220>
   <223> Hif 1 alpha oligomer sequence motif
<400> 54
   gcaagcatcc tg 12
<210> 55
   <211> 16
   <212> DNA
   <213> Artificial
<220>
   <223> Hif 1 alpha oligomer sequence motif
<400> 55
   tggcaagcat cctgta 16
<210> 56
   <211> 16
   <212> DNA
   <213> Artificial
<220>
   <223> LNA oligomer
<220>
   <221> misc feature
   <222> (1) .. (16)
   <223> Phosphorothioate linkage
<220>
   <221> misc feature
   <222> (1)..(3)
   <223> LNA
<220>
   <221> misc_feature
   <222> (13) .. (15)
   <223> LNA
<400> 56
   tggcaagcat cctgta 16
<210> 57
   <211> 14
   <212> DNA
   <213> Artificial
<220>
   <223> LNA oligomer
<220>
   <221> misc_feature
   <222> (1) .. (14)
   <223> Phosphorothioate linkage
<220>
   <221> misc_feature
   <222> (1) .. (2)
   <223> LNA
<220>
   <221> misc_feature
   <222> (12) .. (13)
   <223> LNA
<400> 57
   ggcaagcatc ctgt 14
<210> 58
   <211> 12
   <212> DNA
   <213> Artificial
<220>
   <223> LNA oligomer
<220>
   <221> misc_feature
   <222> (1)..(12)
   <223> Phosphorothioate linkage
<220>
   <221> misc_feature
   <222> (1) .. (2)
   <223> LNA
<220>
   <221> misc_feature
   <222> (11) .. (12)
   <223> LNA
<400> 58
   gcaagcatcc tg 12
<210> 59
   <211> 14
   <212> DNA
   <213> Artificial
<220>
   <223> LNA oligomer
<220>
   <221> misc_feature
   <222> (1) .. (14)
   <223> Phosphorothioate linkage
<220>
   <221> misc_feature
   <222> (1) .. (3)
   <223> LNA
<220>
   <221> misc feature
   <222> (3)..(3)
   <223> 5 methyl cytosine
<220>
   <221> misc_feature
   <222> (12) .. (14)
   <223> LNA
<400> 59
   ggcaagcatc ctgt 14
<210> 60
   <211> 14
   <212> DNA
   <213> Artificial
<220>
   <223> LNA oligomer
<220>
   <221> misc_feature
   <222> (1) .. (14)
   <223> Phosphorothioate linkage
<220>
   <221> misc feature
   <222> (1) .. (2)
   <223> LNA
<220>
   <221> misc feature
   <222> (12)..(14)
   <223> LNA
<400> 60
   ggcaagcatc ctgt 14
<210> 61
   <211> 13
   <212> DNA
   <213> Artificial
<220>
   <223> LNA oligomer
<220>
   <221> misc_feature
   <222> (1) .. (13)
   <223> Phosphorothioate linkage
<220>
   <221> misc_feature
   <222> (1) .. (2)
   <223> LNA
<220>
   <221> misc_feature
   <222> (12) .. (13)
   <223> LNA
<400> 61
   ggcaagcatc ctg 13
<210> 62
   <211> 13
   <212> DNA
   <213> Artificial
<220>
   <223> LNA oligomer
<220>
   <221> misc_feature
   <222> (1) .. (13)
   <223> Phosphorothioate linkage
<220>
   <221> misc_feature
   <222> (1) .. (2)
   <223> LNA
<220>
   <221> misc_feature
   <222> (11) .. (13)
   <223> LNA
<220>
   <221> misc_feature
   <222> (11) .. (11)
   <223> 5 methyl cytosine
<400> 62
   ggcaagcatc ctg 13
<210> 63
   <211> 13
   <212> DNA
   <213> Artificial
<220>
   <223> LNA olgiomer
<220>
   <221> misc_feature
   <222> (1) .. (13)
   <223> Phosphorothioate linkage
<220>
   <221> misc_feature
   <222> (1) .. (2)
   <223> LNA
<220>
   <221> misc_feature
   <222> (2) .. (2)
   <223> 5 methyl cytosine
<220>
   <221> misc_feature
   <222> (11) .. (13)
   <223> LNA
<400> 63
   gcaagcatcc tgt 13
<210> 64
   <211> 13
   <212> DNA
   <213> Artificial
<220>
   <223> LNA oligomer
<220>
   <221> misc_feature
   <222> (1) .. (13)
   <223> Phosphorothioate linkage
<220>
   <221> misc_feature
   <222> (1) .. (2)
   <223> LNA
<220>
   <221> misc_feature
   <222> (2) .. (2)
   <223> 5 methyl cytosine
<220>
   <221> misc_feature
   <222> (12)..(13)
   <223> LNA
<400> 64
   geaageatee tgt 13
<210> 65
   <211> 12
   <212> DNA
   <213> Artificial
<220>
   <223> LNA olgiomer
<220>
   <221> misc_feature
   <222> (1) .. (12)
   <223> Phosphorothioate linkage
<220>
   <221> misc_feature
   <222> (1) .. (1)
   <223> LNA
<220>
   <221> misc_feature
   <222> (11) .. (12)
   <223> LNA
<400> 65
   gcaagcatcc tg 12
<210> 66
   <211> 3958
   <212> DNA
   <213> Homo sapiens
<400> 66
   gtgctgcctc gtctgagggg acaggaggat caccctcttc gtcgcttcgg ccagtgtgtc      60
   gggctgggcc ctgacaagcc acctgaggag aggctcggag ccgggcccgg accccggcga     120
   ttgccgcccg cttctctcta gtctcacgag gggtttcccg cctcgcaccc ccacctctgg     180
   acttgccttt ccttctcttc tccgcgtgtg gagggagcca gcgcttaggc cggagcgagc     240
   ctgggggccg cccgccgtga agacatcgcg gggaccgatt caccatggag ggcgccggcg     300
   gcgcgaacga caagaaaaag ataagttctg aacgtcgaaa agaaaagtct cgagatgcag     360
   ccagatctcg gcgaagtaaa gaatctgaag ttttttatga gcttgctcat cagttgccac     420
   ttccacataa tgtgagttcg catcttgata aggcctctgt gatgaggctt accatcagct     480
   atttgcgtgt gaggaaactt ctggatgctg gtgatttgga tattgaagat gacatgaaag     540
   cacagatgaa ttgcttttat ttgaaagcct tggatggttt tgttatggtt ctcacagatg     600
   atggtgacat gatttacatt tctgataatg tgaacaaata catgggatta actcagtttg     660
   aactaactgg acacagtgtg tttgatttta ctcatccatg tgaccatgag gaaatgagag     720
   aaatgcttac acacagaaat ggccttgtga aaaagggtaa agaacaaaac acacagcgaa     780
   gcttttttct cagaatgaag tgtaccctaa ctagccgagg aagaactatg aacataaagt 840
   ctgcaacatg gaaggtattg cactgcacag gccacattca cgtatatgat accaacagta 900
   accaacctca gtgtgggtat aagaaaccac ctatgacctg cttggtgctg atttgtgaac 960
   ccattcctca cccatcaaat attgaaattc ctttagatag caagactttc ctcagtcgac 1020
   acagcctgga tatgaaattt tcttattgtg atgaaagaat taccgaattg atgggatatg 1080
   agccagaaga acttttaggc cgctcaattt atgaatatta tcatgctttg gactctgatc 1140
   atctgaccaa aactcatcat gatatgttta ctaaaggaca agtcaccaca ggacagtaca 1200
   ggatgcttgc caaaagaggt ggatatgtct gggttgaaac tcaagcaact gtcatatata 1260
   acaccaagaa ttctcaacca cagtgcattg tatgtgtgaa ttacgttgtg agtggtatta 1320
   ttcagcacga cttgattttc tcccttcaac aaacagaatg tgtccttaaa ccggttgaat 1380
   cttcagatat gaaaatgact cagctattca ccaaagttga atcagaagat acaagtagcc 1440
   tctttgacaa acttaagaag gaacctgatg ctttaacttt gctggcccca gccgctggag 1500
   acacaatcat atctttagat tttggcagca acgacacaga aactgatgac cagcaacttg 1560
   aggaagtacc attatataat gatgtaatgc tcccctcacc caacgaaaaa ttacagaata 1620
   taaatttggc aatgtctcca ttacccaccg ctgaaacgcc aaagccactt cgaagtagtg 1680
   ctgaccctgc actcaatcaa gaagttgcat taaaattaga accaaatcca gagtcactgg 1740
   aactttcttt taccatgccc cagattcagg atcagacacc tagtccttcc gatggaagca 1800
   ctagacaaag ttcacctgag cctaatagtc ccagtgaata ttgtttttat gtggatagtg 1860
   atatggtcaa tgaattcaag ttggaattgg tagaaaaact ttttgctgaa gacacagaag 1920
   caaagaaccc attttctact caggacacag atttagactt ggagatgtta gctccctata 1980
   tcccaatgga tgatgacttc cagttacgtt ccttcgatca gttgtcacca ttagaaagca 2040
   gttccgcaag ccctgaaagc gcaagtcctc aaagcacagt tacagtattc cagcagactc 2100
   aaatacaaga acctactgct aatgccacca ctaccactgc caccactgat gaattaaaaa 2160
   cagtgacaaa agaccgtatg gaagacatta aaatattgat tgcatctcca tctcctaccc 2220
   acatacataa agaaactact agtgccacat catcaccata tagagatact caaagtcgga 2280
   cagcctcacc aaacagagca ggaaaaggag tcatagaaca gacagaaaaa tctcatccaa 2340
   gaagccctaa cgtgttatct gtcgctttga gtcaaagaac tacagttcct gaggaagaac 2400
   taaatccaaa gatactagct ttgcagaatg ctcagagaaa gcgaaaaatg gaacatgatg 2460
   gttcactttt tcaagcagta ggaattggaa cattattaca gcagccagac gatcatgcag 2520
   ctactacatc actttcttgg aaacgtgtaa aaggatgcaa atctagtgaa cagaatggaa 2580
   tggagcaaaa gacaattatt ttaataccct ctgatttagc atgtagactg ctggggcaat 2640
   caatggatga aagtggatta ccacagctga ccagttatga ttgtgaagtt aatgctccta 2700
   tacaaggcag cagaaaccta ctgcagggtg aagaattact cagagctttg gatcaagtta 2760
   actgagcttt ttcttaattt cattcctttt tttggacact ggtggctcac tacctaaagc 2820
   agtctattta tattttctac atctaatttt agaagcctgg ctacaatact gcacaaactt 2880
   ggttagttca atttttgatc ccctttctac ttaatttaca ttaatgctct tttttagtat 2940
   gttctttaat gctggatcac agacagctca ttttctcagt tttttggtat ttaaaccatt 3000
   gcattgcagt agcatcattt taaaaaatgc acctttttat ttatttattt ttggctaggg 3060
   agtttatccc tttttcgaat tatttttaag aagatgccaa tataattttt gtaagaaggc 3120
   agtaaccttt catcatgatc ataggcagtt gaaaaatttt tacacctttt ttttcacatt 3180
   ttacataaat aataatgctt tgccagcagt acgtggtagc cacaattgca caatatattt 3240
   tcttaaaaaa taccagcagt tactcatgga atatattctg cgtttataaa actagttttt 3300
   aagaagaaat tttttttggc ctatgaaatt gttaaacctg gaacatgaca ttgttaatca 3360
   tataataatg attcttaaat gctgtatggt ttattattta aatgggtaaa gccatttaca 3420
   taatatagaa agatatgcat atatctagaa ggtatgtggc atttatttgg ataaaattct 3480
   caattcagag aaatcatctg atgtttctat agtcactttg ccagctcaaa agaaaacaat 3540
   accctatgta gttgtggaag tttatgctaa tattgtgtaa ctgatattaa acctaaatgt 3600
   tctgcctacc ctgttggtat aaagatattt tgagcagact gtaaacaaga aaaaaaaaat 3660
   catgcattct tagcaaaatt gcctagtatg ttaatttgct caaaatacaa tgtttgattt 3720
   tatgcacttt gtcgctatta acatcctttt tttcatgtag atttcaataa ttgagtaatt 3780
   ttagaagcat tattttagga atatatagtt gtcacagtaa atatcttgtt ttttctatgt 3840
   acattgtaca aatttttcat tccttttgct ctttgtggtt ggatctaaca ctaactgtat 3900
   tgttttgtta catcaaataa acatcttctg tggaccagga aaaaaaaaaa aaaaaaaa 3958
<210> 67
   <211> 16
   <212> DNA
   <213> Artificial
<220>
   <223> anti-Hiflalpha oligomer sequence motif
<400> 67
   tggcaagcat cctgta 16
<210> 68
   <211> 16
   <212> DNA
   <213> Artificial
<220>
   <223> LNA oligomer
<220>
   <221> misc_feature
   <222> (1)..(16)
   <223> Phosphorothioate linkage
<220>
   <221> misc_feature
   <222> (1) .. (3)
   <223> LNA
<220>
   <221> misc_feature
   <222> (13) .. (15)
   <223> LNA
<400> 68
   tggcaagcat cctgta 16
<210> 69
   <211> 16
   <212> DNA
   <213> Artificial
<220>
   <223> Anti-Hiflalpha LNA oligomer
<220>
   <221> misc_feature
   <222> (1) .. (2)
   <223> Phosphorothioate linkage
<220>
   <221> misc_feature
   <222> (1) .. (3)
   <223> LNA
<220>
   <221> misc_feature
   <222> (3) .. (12)
   <223> Phosphorothioate linkages
<220>
   <221> misc_feature
   <222> (13) .. (16)
   <223> Phosphorothioate linkages
<220>
   <221> misc_feature
   <222> (13) .. (15)
   <223> LNA
<400> 69
   tggcaagcat cctgta 16